# FASCICULE DE BREVET EUROPEEN

(11) **EP 0 901 463 B1**
(45) Date de publication et mention de la délivrance du brevet: **14.06.2000**
(21) Numéro de dépôt: 98908156.7
(22) Date de dépôt: 10.02.1998
(51) Int. Cl.: C07C 237/08, A61K 31/21, C07C 229/16, C07H 21/00, C12N 15/63, C12N 5/10

(54) **COMPOSES GLYCEROLIPIDIQUES UTILES POUR LE TRANSFERT D'UNE SUBSTANCE ACTIVE DANS UNE CELLULE CIBLE**
GLYCEROLIPIDE VERBINDUNGEN UND IHRE ANWENDUNG ZUM TRANSPORT EINER AKTIVEN SUBSTANZ IN EINE ZIELZELLE
GLYCEROLIPIDIC COMPOUNDS USED FOR THE TRANSFER OF AN ACTIVE SUBSTANCE INTO A TARGET CELL

(30) Priorité: 10.02.1997 FR 9701475; 12.12.1997 FR 9715805
(43) Date de publication de la demande: 17.03.1999
(73) Titulaire: TRANSGENE S.A., 67000 Strasbourg (FR)
(72) Inventeur: BISCHOFF, Rainer, F-67400 Illkirch-Graffenstaden (FR); HEISSLER, Denis, F-67201 Eckbolsheim (FR); NAZIH, Abdesslame, F-67000 Strasbourg (FR)
(74) Mandataire: Warcoin, Jacques
(86) Numéro de dépôt international: FR9800250
(87) Numéro de publication internationale: WO9834910

(56) Documents cités:
- EP-A- 0 685 234
- EP-A- 0 685 457
- WO-A-94/05624
- DE-A- 19 521 412
- EDWARD R. LEE ET AL: "HUMAN GENE THERAPY, Vol. 7, pages 1701 à 1717

## Description

La présente invention concerne de nouveaux composés glycérolipidiques et de nouvelles compositions les comprenant. Plus particulièrement, la présente invention concerne l'utilisation desdits composés ou desdites compositions pour préparer un vecteur de transfert d'une substance active, notamment thérapeutiquement comportant des charges négatives, notamment un polynucléotide, dans une cellule cible, particulièrement une cellule de vertébré, et plus particulièrement de mammifère.

Le transfert de gène dans une cellule donnée est la base même de la thérapie génique. Cette nouvelle technologie dont le champ d'application est vaste, permet d'envisager le traitement de maladies graves pour lesquelles les alternatives thérapeutiques classiques sont peu efficaces, voire inexistantes, et concerne aussi bien les maladies d'origine génétique (hémophilie, mucoviscidose, myopathie, ...) qu'acquises (cancer, SIDA,...).

Au cours des 30 dernières années, de nombreux outils ont été mis au point permettant l'introduction de divers gènes hétérologues dans des cellules, notamment des cellules de mammifère. Ces différentes techniques peuvent être divisées en deux catégories. La première catégorie concerne les techniques physiques telles que la microinjection, l'électroporation ou le bombardement particulaire qui, bien qu'efficaces, sont largement limitées à des applications *in vitro* et dont la mise en oeuvre est lourde et délicate. La seconde catégorie fait appel à des techniques relatives à la biologie moléculaire et cellulaire pour lesquelles le gène à transférer est associé à un vecteur de nature biologique ou synthétique qui favorise l'introduction dudit matériel.

Actuellement, les vecteurs les plus efficaces sont des vecteurs viraux, en particulier adénoviraux ou rétroviraux. Les techniques développées reposent sur les propriétés naturelles dont disposent ces virus pour traverser les membranes cellulaires, échapper à la dégradation de leur matériel génétique et faire pénétrer leur génome dans le noyau. Ces virus ont déjà fait l'objet de nombreuses études et certains d'entre eux sont d'ores et déjà employés à titre expérimental comme vecteurs de gènes chez l'homme en vue par exemple d'une vaccination, d'une immunothérapie ou d'une thérapie visant à suppléer une déficience génétique. Toutefois, cette approche virale présente de nombreuses limitations, notamment en raison de la capacité de clonage restreinte dans le génome viral, des risques de dissémination dans l'organisme hôte et dans l'environnement des particules virales infectieuses produites, du risque de mutagénèse artéfactuel par insertion dans la cellule hôte dans le cas des vecteurs rétroviraux, et de la forte induction des réponses immunitaire et inflammatoire in vivo lors du traitement thérapeutique, limitant considérablement le nombre d'administrations pouvant être envisagées (Mc Coy et al, 1995, Human Gene Therapy, 6, 1553-1560 ; Yang et al., 1996, Immunity, 1, 433-442). Ces nombreux inconvénients, notamment dans le cadre d'un usage chez l'homme, ont conduit plusieurs équipes à développer des systèmes alternatifs de transfert de polynucléotides.

Plusieurs méthodes non-virales sont disponibles à l'heure actuelle. Citons pour exemple la coprécipitation au phosphate de calcium, l'utilisation de récepteurs mimant les systèmes viraux (pour une revue voir Cotten et Wagner, 1993, Current Opinion in Biotechnology, 4, 705-710), ou l'utilisation de polymères tels que le polyamidoamine (Haensler et Szoka, 1993, Bioconjugate Chem., 4, 372-379), ou de polymère tels que ceux présentés dans WO 95/24221 décrivant l'utilisation de polymères dendritiques, le document WO 96/02655 décrivant l'utilisation de polyéthylène imine, ou de polypropylène imine et les documents US-A- 5 595 897 et FR 2 719 316 décrivant l'utilisation de conjugués de polylysine. D'autres techniques non virales s'appuient sur l'utilisation de liposomes dont l'intérêt à titre d'agent permettant l'introduction, à l'intérieur de cellules, de certaines macromolécules biologiques, telles que par exemple l'ADN, l'ARN, les protéines ou certaines substances pharmaceutiquement actives a été largement décrit dans la littérature. A cette fin, plusieurs équipes ont déjà proposé l'utilisation de lipides cationiques qui présentent une forte affinité à l'égard des membranes cellulaires et/ou des acides nucléiques. En effet, bien qu'il ait été montré, dans le cas des acides nucléiques, que ce type de macromolécule est capable de traverser la membrane plasmatique de certaines cellules *in vivo* (WO 90/11092), il n'en demeure pas moins que l'efficacité de la transfection observée est encore très limitée, en raison notamment de la nature polyanionique des acides nucléiques qui prévient leur passage au travers ce la membrane cellulaire, présentant elle-même une charge apparente nette négative. Dés 1989 (Felgner et al., Nature, 337, 387-388) les lipides cationiques ont été présentés comme des molécules intéressantes pour favoriser l'introduction de grosses molécules anioniques, telles que les acides nucléiques, dans certaines cellules. Ces lipides cationiques sont capables de se complexer aux molécules anioniques tendant ainsi à neutraliser les charges négatives desdites molécules et à favoriser leur rapprochement vers les cellules. De nombreuses équipes ont déjà développé différents lipides cationiques. A titre d'exemples, on peut citer le DOTMA (Felgner et al., 1987, PNAS, 84, 7413-7417), le DOGS ou Transfectam™ (Behr et al., 1989, PNAS, 86, 6982-6986), le DMRIE et le DORIE (Felgner et al., 1993, Methods 5, 67-75), le DC-CHOL (Gao et Huang, 1991, BBRC, 179, 280-285), le DOTAP™ (McLachlan et al., 1995, Gene Therapy, 2,674-622) ou la Lipofectamine™, ainsi que ceux décrits dans les demandes de brevet WO9116024 ou W09514651.

Plus particulièrement, la demande W09405624 décrit des lipides cationiques de formule : 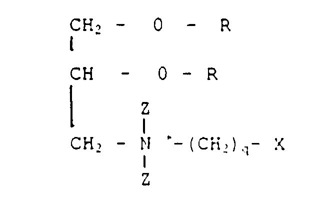 dans laquelle les radicaux R sont notamment des radicaux octadécènyle, les radicaux Z sont des radicaux alkyle C₁-C₁₃ ou -C(=O)-(C₁-C₁₃) alkyle ou alcyle, q est un nombre entier de 1 à 6, X est notamment une courte chaîne polyamine, telle que la spermine, la spermidine, une carboxyspermine ou une polylysine.

Les demandes EP 685457 et EP 685234 décrivent notamment des composés cationiques de formule : 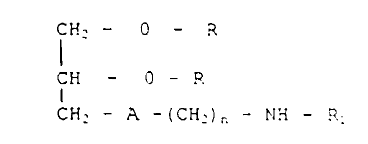 dans laquelle R est en particulier une chaîne hydrocarbonée de 10 à 30 atomes de carbone, saturée ou non, A peut notamment être choisi parmi les groupes -O-(C=O)- et -NH-(C=O)-,n varie de 0 à 4 et R₁ est un alkyle ou une courte chaîne aminoalkyle dans laquelle l'amine primaire est substituée par un alkyle de 2 à 8 atomes de carbone. Ces composés présentent une faible activité hémolytique et permettent *in vitro* d'introduire dans des cellules HelaS3 un ARN double brin pouvant agir comme agent d'inhibition de la croissance.

La demande DE 19521412 décrit des composés comportant eux aussi une partie non polaire et une partie polaire de formule : 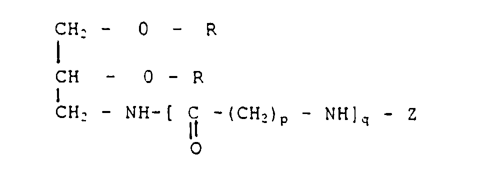 dans laquelle p varie de 1 à 6, q varie de 0 à 2, R est soit C(=O)-C1-23 ou C1-23, saturé ou non, et Z est un peptide, un acide aminé ou une structure aminée branchée. Ces composés cationiques permettent la transfection *in vitro* de cellules en culture.

Toutefois, plusieurs travaux (à titre d'exemples, voir Mahato et al., J. Pharm. Sci., 1995, 84, 1267-1271, Thierry et al., 1995, P.N.A.S, 92, 9742-9746) ont mis en évidence que l'efficacité du transfert à l'intérieur des cellules de la macromolécule anionique pouvait varier en fonction notamment de l'interaction entre les complexes et les membranes cellulaires, de la cellule considérée, de la composition lipidique des composés cationiques, de la taille des complexes formés avec les molécules anioniques et plus particulièrement du rapport de charges positives et négatives des différents composants dudit complexe. Les mécanismes qui permettent en particulier l'interaction des complexes avec les membranes cellulaires et le transfert des complexes à l'intérieur de la cellule sont encore largement méconnus et les chercheurs procèdent dans leurs travaux selon une approche fortement empirique. Il est par conséquent souhaitable de proposer d'autres lipides cationiques, ayant éventuellement des propriétés améliorées ou différentes de ceux déjà décrits.

Le déposant a aujourd'hui identifié de nouveaux composés glycérolipidiques, qui peuvent se présenter sous forme cationique, utiles notamment pour transférer une substance active comportant des charges négatives, notamment un polynucléotide, à l'intérieur d'une cellule cible, dont on peut envisager l'utilisation notamment *in vivo* dans le cadre d'une thérapie génique.

Ainsi, la présente invention a tout d'abord pour objet un composé de formule : 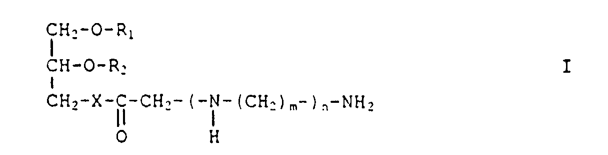 dans laquelle :
R₁, R₂, identiques ou différents, sont des radicaux de 6 à 23 atomes de carbone (notés C₆-C₂₃) alkyle ou alcényle, linéaires ou ramifiés ou des radicaux -C(=O)-(C₆-C₂₃) alkyle ou-C(=O)-(C₆-C₂₃) alcényle, ou plus particulièrement -C(=O)-(C₁₂-C₂₀) alkyle ou -C(=O)-(C₁₂-C₂₀) alcényle, linéaires ou ramifiés, des radicaux aryle, des radicaux cycloalkyle, des radicaux fluoroalkyle, des groupements oxyéthylène ou oxyméthylène éventuellement répétés, linéaires ou ramifiés, éventuellement substitués,
X est l'atome d'oxygène ou un radical amino -NR₃, R₃ étant un atome d'hydrogène ou un radical alkyle de 1 à 4 atomes de carbone,
n est un nombre entier positif de 1 à 6, de manière préférée de 2 à 4,
m est un nombre entier positif de 1 à 6, de manière préférée de 2 à 4, et lorsque n > 1, m peut être identique ou différent dudit n.

Par le terme «alcényle», on entend indiquer que la chaîne carbonée dont il est question peut comprendre une ou plusieurs double(s) liaison(s) le long de ladite chaîne.

Selon un cas particulier de l'invention, lesdits composés sont caractérisés en ce que R1 ou/et R2 sont fluorés, c'est à dire qu'au moins un carbone de la chaîne polycarbonée est substitué par un groupe fluoré. Des exemples de telles molécules sont proposés à l'exemple N. Dans ce cas particulier, le nombre d'atomes de carbone fluorés sur chaque chaîne R₁ ou R₂ peut varier de 1 à 12, et plus particulièrement de 4 à 8, et est préférentiellement 4. Selon un cas avantageux, R1 et/ou R2 sont des radicaux alkyles de 15 atomes de carbonne et le nombre d'atomes de carbone fluorés est 4 pour chacune de chaînes R₁ et R₂ concernée. Le nombre de groupes fluorés présents sur les chaînes R₁ et/ou R₂ peut notamment varier de 1 à 23, plus particulièrement de 9 à 17 et préférentiellement est 9.

Les composés selon l'invention peuvent en outre être substitués. De telles substitutions peuvent notamment consister en une molécule de marquage (voir molécules de marquage dans US 4711955) qui permet par exemple de visualiser la distribution des composés ou des complexes les renfermant après administration *in vitro* ou *in vivo,* une molécule de ciblage cellulaire, une molécule d'ancrage. L'invention concerne par conséquent également un composé tel que présenté ci-dessus conjugué à un ou plusieurs éléments de ciblage par l'intermédiaire d'au moins a) un des atomes de carbone, en particulier choisis parmi ceux présents sur les groupements R₁, R₂ et/ou R₃ ou b) un des atomes d'azote secondaire ou primaire de la chaîne polyamine. De tels éléments peuvent permettre le ciblage vers un type cellulaire particulier, faciliter la pénétration à l'intérieur de la cellule, la lyse des endosomes ou encore le transport intracellulaire et sont largement décrits dans la littérature. Il peut s'agir par exemple de tout ou partie de sucres, de peptides (peptide GRP, Gastrin Releasing Peptide, par exemple), d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogéniques, de peptides de localisation nucléaire, ou d'une combinaison de tels composés. On peut citer plus particulièrement les résidus galactosyl permettant de cibler le récepteur asyaloglycoprotéique à la surface des cellules hépatiques, le peptide fusogénique INF-7 dérivé de la sous-unité HA-2 de l'hémagglutinine du virus influenza (Plank et al. 1994, J. Biol. Chem. *269,* 12918-12924) ou d'un signal de localisation nucléaire dérivé de l'antigène T du virus SV40 (Lanford et Butel, 1984, Cell 37, 801-813) ou de la protéine EBNA-1 du virus Epstein Barr (Ambinder et al., 1991, J. Virol. 65, 1466-1478).

De tels conjugués peuvent être aisément obtenus selon les techniques largement décrites dans la littérature, et plus particulièrement par couplage chimique, notamment en utilisant des groupes protecteurs tels que trifluoroacétyle ou Fmoc ou Boc sur la polyamine. La déprotection sélective d'un groupe protecteur permet alors de coupler l'élément de ciblage, et l'on déprotège ensuite le glycérolipide. Il convient toutefois d'indiquer que la substitution des groupes non réactifs tels que les atomes de carbone dans les groupements CH ou CH2, sera réalisée en cours de synthèse des composés de l'invention selon des méthodes connues de l'homme du métier ; tandis que les groupes réactifs, tels que les amines primaires ou secondaires, peuvent faire l'objet de substitutions sur les glycérolipides de l'invention néosynthétisés.

Selon un cas avantageux de l'invention, ledit composé est sous forme cationique, c'est à dire qu'il est sous forme protonée par fixation d'un proton sur un ou plusieurs atomes d'azote présents sur la chaîne polyamine. Dans ce cas, ledit glycérolipide cationique est associé à un ou plusieurs anions biologiquement acceptables, tels que par exemple l'anion trifluoroacétate, halogénure, monométhylsulfate, acétate ou phosphate, iodure, chlorure, bromure... Il est également possible d'obtenir des composés sous forme cationique par substitution des amines par exemple avec un radical méthyl, éthyl, ...

Les composés selon la présente invention comportent de 2 à 7 charges positives, plus particulièrement de 3 à 5, et préférentiellement 5. Il a été montré que l'affinité d'une polyamine pour l'ADN dépend notamment du nombre de fonctions amines présentes sur ladite polyamine (Braulin, W. H., Strick, T. J., and Record, M. T., Jr. (1982) Biopolymers 21, 1301-1314). Par ailleurs, suite à leur pénétration dans la cellule par endocytose, les complexes formés entre un ADN et un composé lipidique cationique sont localisés dans les endosomes dans lesquels l'ADN peut être dégraàé sous l'action de nucléases pH-dépendantes. Pour contrer ce phénomène qui affecte l'efficacité de la transfection, il est possible d'utiliser des agents lysosomotropiques, tels que par exemple la chloroquine, dont la capacité tampon à pH 5,5 permet d'observer une amélioration de la transfection. Toutefois, l'efficacité de tels composés n'est pas systématique, notons à titre d'exemples négatifs les cas de la polyéthyleimine (PEI), des lipospermines ou des dendrimères de polyamidoamine (PAMAM). Par ailleurs, l'utilisation de chloroquine à forte dose peut présenter un risque toxique. Selon l'invention, les composés possèàent une tête polyaminée qui permet d'obtenir un effet similaire à celui des molécules lysosomotropiques. Un intérêt particulier desdits composés pourrait être, par conséquent, d'éviter l'utilisation de la chloroquine pour les applications *in vivo*.

Selon un mode de réalisation préféré de l'invention, ledit composé est choisi dans le groupe consistant en les formules suivantes:

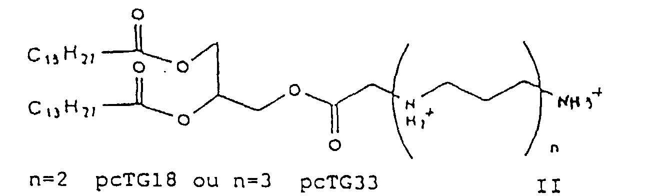

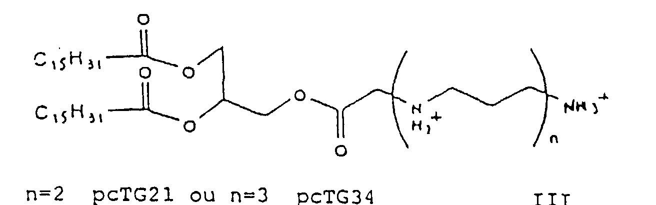

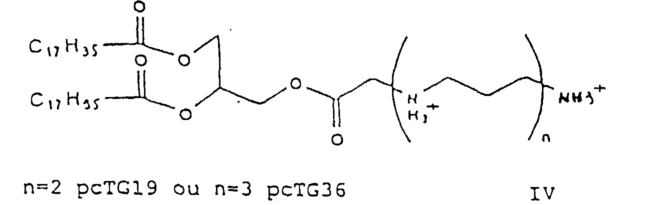

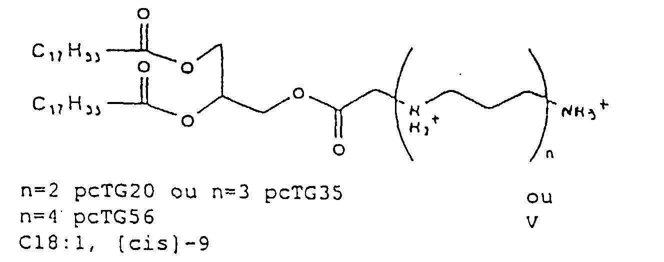

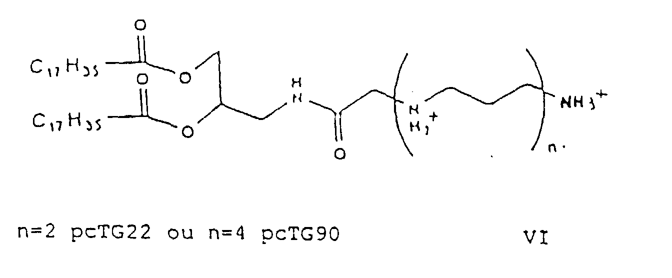

Ces composés cationiques peuvent par exemple être préparés par réaction d'un composé de formule : 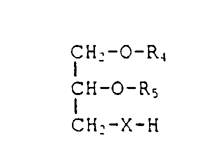 dans laquelle:
R₄, R₅ sont des groupes protecteurs, formant notamment ensemble un radical isopropylidène,
X a la même signification que dans la formule I, avec un acide de formule : 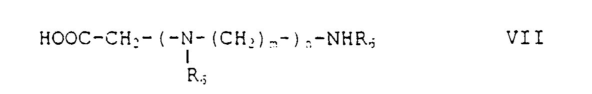
m et n ayant la même signification que dans la formule I,
R₆ étant un groupe protecteur, notamment le t-butoxycarbonyle (BOC).

Les fonctions O-R₄, -O-R₅ sont ensuite déprotégées pour fixer par estérification ou éthérification les radicaux R₁ et R₂ de manière connue.

Le composé obtenu est déprotégé en présence d'acide trifluoroacétique. L'acide de formule VII est préparé de manière connue.

Dans le cas des composés de l'invention pour lesquels m = 3, n = 2 ou 3, on se référera aux exemples indiqués ci-après pour connaître les modalités pratiques de synthèse. Les procédés décrits sont applicables en général aux synthèses des composés selon l'invention moyennant des adaptations à la portée de l'homme du métier. Toutefois, les composés de l'invention ne sauraient se limiter à ceux obtenus par les modes de préparation décrits précédemment.

Selon un autre aspect, l'invention concerne également une composition comprenant au moins un composé tel que décrit ci-dessus et éventuellement au moins un adjuvant susceptible d'améliorer la formation de complexe entre undit composé et une substance active, ou d'améliorer le fonctionnement de ces complexes vis-à-vis de la cellule.

De manière préférée, un tel adjuvant sera un lipide neutre ou zwitterionique, qui par exemple est ou dérive d'un triglycéride, un diglycéride, le cholestérol (voir par exemple US 5 438 044), en particulier, un lipide neutre ou zwitterionique qui est ou qui dérive d'une phosphatidyl éthanolamine (PE), phosphatidylcholine, phosphocholine, sphyngomyéline, céramide ou cérébroside. De manière avantageuse, on choisira la dioleoylphosphatidyl éthanolamine (DOPE).

Le rapport en poids entre le composé de l'invention et le lipide neutre ou zwitterionique est généralement compris entre 0, 1 et 10, étant entendu que ce rapport peut varier selon la nature des composants considérés. L'homme du métier dispose des connaissances suffisantes pour permettre ces adaptations mineures. Il est également possible d'utiliser un mélange de lipides neutres et/ou zwitterioniques.

L' invention concerne en outre un complexe comprenant au moins un composé ou au moins une composition tels que décrits précédemment et au moins une substance active, notamment thérapeutiquement, comportant au moins une charge négative. Selon une variante de l'invention, ledit complexe peut en outre renfermer un ou plusieurs amphiphiles cationiques tels que ceux décrits dans la littérature dont des exemples ont été proposés plus haut. En général, on utilisera des substances thérapeutiquement actives qui peuvent être mises en oeuvre notamment dans le cadre de la thérapie génique.

Selon un mode particulier de réalisation, ladite substance active est choisie parmi les acides nucléiques et les protéines. De préférence, la substance active du complexe selon l'invention est un polynucléotide, ledit composé ou ladite composition permettant alors d'améliorer le pouvoir transfectant du polynucléotide dans une cellule.

Par «polynucléotide», on entend désigner un fragment d'ADN et/ou d'ARN, double brin ou simple brin, linéaire ou circulaire, naturel isolé ou de synthèse, désignant un enchaînement précis de nucléotides, modifiés ou non (voir à titre d'exemple US 5525711), marqués ou non (voir, par exemple, US 4711955 ou EP 302175), permettant de définir un fragment ou une région d'un acide nucléique sans limitation de taille. Par polynucléotide on entend désigner notamment un cADN, un ADN génomique, un ADN plasmidique, un ARN messager, un ARN antisens, un ribozyme, un ARN de transfert, un ARN ribosomique, ou un ADN codant pour de tels ARN. «Polynucléotide» ou «acide nucléique» sont des termes synonymes dans le cadre de la présente demande.

Selon un mode de réalisation particulier de l'invention, ledit polynucléotide comprend un gène d'intérêt et des éléments permettant l'expression dudit gène d'intérêt. Dans ce mode de réalisation, ledit polynucléotide est avantageusement sous forme de plasmide. Les éléments permettant l'expression sont l'ensemble des éléments permettant la transcription dudit fragment d'ADN en ARN (ARN antisens ou ARNm) et la traduction de l'ARNm en polypeptide. Il s'agit notamment des séquences promotrices et/ou des séquences de régulation efficaces dans ladite cellule, et éventuellement les séquences requisent pour permettre l'excrétion ou l'expression à la surface des cellules cibles dudit polypeptide. A titre d'exemple, on mentionnera les promoteurs tels que les promoteurs des virus RSV, MPSV, SV40, CMV ou 7.5k, du virus de la vaccine, les promoteurs du gêne codant pour la créatine kinase musculaire, pour l'actine, pour le surfactant pulmonaire. Il est en outre possible de choisir une séquence promotrice spécifique d'un type cellulaire donné, ou activable dans des conditions définies. La littérature procure un grand nombre d'informations relatives à de telles séquences promotrices. Par ailleurs, ledit polynucléotide peut comprendre au moins deux séquences, identiques ou différentes, présentant une activité de promoteur transcriptionnel et/ou au moins deux séquences codantes d'ADN, identiques ou différentes, situées l'une par rapport à l'autre de manière contiguë, éloignée, dans le même sens ou en sens inverse, pour autant que la fonction de promoteur transcriptionnel ou la transcription desdites séquences ne soit pas affectée. De même, dans ce type de construction d'acide nucléique, il est possible d'introduire des séquences nucléiques «neutres» ou introns qui n'affectent pas la transcription et sont épissées avant l'étape de traduction. De telles séquences et leurs utilisations sont décrites dans la littérature. Ledit polynucléotide pourra également renfermer des séquences requises pour le transport intracellulaire, pour la réplication et/ou pour l'intégration. De telles séquences sont bien connues de l'homme de l'art. Par ailleurs, les polynucléotides selon la présente invention peuvent également être des polynucléotides modifiés de sorte qu'il ne leur est pas possible de s'intégrer dans le génôme de la cellule cible ou des polynucléotides stabilisés à l'aide d'agents, tels que par exemple la spermine.

Dans le cadre de la présence invention, le polynucléotide peut être homologue ou hétérologue à la cellule cible. Il peut être avantageux d'utiliser un polynucléotide qui code pour tout ou partie d'un polypeptide, notamment un polypeptide présentant une activité thérapeutique ou prophylactique, et plus particulièrement une activité immunogène de type cellulaire ou humorale. Le terme polypeptide s'entend sans restriction quant à sa taille ou son degré de modification (par exemple de glycosylation). On peut à titre d'exemples citer les gènes codant pour un enzyme, une hormone, une cytokine, un récepteur membranaire, un polypeptide structural, un polypeptide formant un canal membranaire, un polypeptide de transport, une molécule d'adhésion, un ligand, un facteur de régulation de la transcription, de la traduction, de la réplication, de la stabilisation des transcripts, ou un anticorps, tels que par exemple le gène codant pour la protéine CFTR, la dystrophine, le facteur VIII ou IX, E6/E7 de HPV, MUC1, BRAC1, l'interféron β, l'interféron γ, l'interleukine (IL)2, l'IL-4, l'IL-6, l'IL-7, l'IL-12, le facteur nécrosant de tumeur (TNF) de type alpha, le GM-CSF (Granulocyte Macrophage Colony Stimulating Factor), le gène tk du virus Herpes Simplex de type 1 (HSV-1), le gène associé au rétinoblastome ou p53 ou tout ou partie d'immunoglobulines, telles que les fragments F(ab)₂, Fab', Fab ou les anti-idiotypes (US 4,699,880). Bien entendu, cette liste n'est pas limitative et d'autres gènes peuvent être employés.

Selon un mode de réalisation préféré, les complexes selon l'invention sont de faible taille (inférieure à 500 nm, avantageusement à 200 nm et de préférence à 100 nm).

Par ailleurs, les expériences de transfection réalisées montrent qu'avantageusement le rapport en poids du composé lipidique selon l'invention audit polynucléotide est de 0,01 à 100. Le rapport optimal se situe de 0,05 à 10.

L'invention concerne également un procédé de préparation des complexes composés cationiques/substances actives comportant au moins une charge négative, ledit procédé étant caractérisé en ce que l'on met en présence un ou plusieurs composés ou compositions selon l'invention avec une ou plusieurs substances actives comportant au moins une charge négative et en ce que l'on récupère ledit complexe, éventuellement après une étape de purification.

Dans un premier temps, selon une première variante, un ou plusieurs composés cationiques sont mis en solution avec une quantité appropriée de solvant ou mélange de solvants miscibles dans l'eau, notamment l'éthanol, le diméthylsulfoxide (DMSO), ou de façon préférée un mélange éthanol/DMSO 1:1 (v:v), de manière à former des agrégats lipidiques selon une méthode connue décrite par exemple dans la demande de brevet WO-A-9603977, ou selon une seconde variante, sont mis en suspension avec une quantité appropriée d'une solution de détergent comme un octylglucoside tel que le n-octyl β-D-glucopyranoside, ou le 6-0-(N-heptylcarbomoyl)-méthyl-a-D-glucopyranoside.

La suspension peut ensuite être mélangée avec une solution de substance active comportant des charges négatives.

Dans le cas où l'on souhaite qu'un lipide neutre ou zwitterionique soit présent dans le complexe final, on forme, de manière connue, préalablement à la mise en solution dans le solvant miscible à l'eau ou dans la solution de détergent, un film avec un mélange renfermant undit composé cationique et undit lipide neutre ou zwitterionique, tel que par exemple la DOPE.

Une des caractéristiques importantes du procédé réside dans le choix du rapport entre les charges positives du lipide cationique et les charges négatives de la substance active.

Sans vouloir être limité par un rapport spécifique, on choisira des quantités des différentes charges de manière à ce que le rapport entre le nombre de charges positives du composé ou de la composition cationique et le nombre de charges négatives de la substance active soit compris entre 0,05 et 20, notamment entre 0,1 et 15 et de préférence entre 5 et 10.

Le calcul pour arriver à un tel rapport prendra en considération les charges négatives portées par la substance active et on ajustera la quantité de composé nécessaire pour satisfaire au rapport indiqué ci-dessus. Les quantités et les concentrations pour les autres composants sont ajustées en fonction de leurs masses molaires respectives et de leur nombre de charges positives et/ou négatives.

Ce rapport de charges constitue également une caractéristique avantageuse du complexe selon l'invention.

Dans le cas de la seconde variante et de manière optionnelle, on peut procéder à une dialyse subséquente pour réduire le détergent et récupérer les complexes. Le principe d'une telle méthode est par exemple décrit par Hofland et al. (1996, PNAS 93, p 7305-7309) et dans le chapitre II du document Philippot et al. (G. Gregoriadis, 81-89, CRC Press 1993).

On a montré que la première variante conduit à d'excellents résultats en terme de taille des complexes obtenus.

Selon une troisième variante, un ou plusieurs composés ou compositions cationiques, sont mis en suspension dans un tampon puis la suspension est soumise à une sonication jusqu'à homogénéité visuelle. La suspension lipidique est ensuite extrudée au travers de deux membranes microporeuses sous pression appropriée. La suspension lipidique est alors mélangée avec une solution de substance active comportant des charges négatives. Cette technique dite de sonication-extrusion est bien connue dans l'art.

L'utilisation d'un lipide neutre ou zwitterionique, tel que la DOPE, peut s'avérer avantageuse pour l'obtention de complexes de faible taille (inférieure à 200 nm, de préférence inférieure à 100 nm).

Les caractéristiques des complexes formés peuvent être évaluées par plusieurs moyens permettant de déterminer, par exemple :
- l'état de complexation avec la substance active, notamment par recherche des acides nucléiques libres par électrophorése sur gel d'agarose dans le cas où les substances sont des acides nucléiques,
- la taille des particules par diffusion quasi élastique de la lumière,
- l'absence de précipitation à long terme.

La présente invention a également pour objet les complexes obtenus par la mise en oeuvre des procédés énumérés ci-dessus.

L'invention concerne aussi l'utilisation d'un composé, d'une composition ou d'un complexe selon l'invention pour transférer au moins une substance active, notamment thérapeutiquement, en particulier un acide nucléique, dans des cellules cibles, *in vitro, ex vivo* ou *in vivo,* plus particulièrement *in vivo.*

Par «cellules cibles» selon l'invention, on entend les cellules procaryotes, les cellules de levure et les cellules eucaryotes, les cellules de plantes, les cellules humaines ou animales, et en particulier les cellules de mammifère. Il convient par ailleurs de citer les cellules cancéreuses. In *vivo,* l'invention peut être appliquée au niveau de l'espace interstitiel ou luminal de tissus tels que le poumon, la trachée, la peau, le muscle, le cerveau, le foie, le coeur, la rate, la moelle osseuse, le thymus, la vessie, la lymphe, le sang, le pancreas, l'estomac, le rein, les ovaires, les testicules, le rectum, le système nerveuse périphérique ou central, les yeux, les organes lymphoïdes, les cartilages, l'endothélium. Selon un choix avantageuses de l'invention, la cellule cible sera une cellule musculaire, une cellule souche hématopoïétique ou encore une cellule des voies aériennes, plus particulièrement une cellule trachéale ou pulmonaire, et avantageusement une cellule de l'épithélium respiratoire.

L'invention concerne également un procédé de transfert in vitro d'une substance thérapeutiquement active dans une cellule cible, selon lequel on met en contact ladite cellule avec un complexe selon l'invention.

Les complexes selon l'invention sont utilisables à titre de médicament, à but curatif, préventif ou vaccinal. C'est pourquoi l'invention a également pour objet les complexes de l'invention à titre de médicament à but curatif, préventif ou vaccinal. De tels complexes peuvent être mis en oeuvre dans une méthode de traitement thérapeutique consistant à transférer au moins une substance thérapeutiquement active, notamment un polynucléotide, dans des cellules cibles, notamment une cellule de mammifère, et plus précisemment une cellule musculaire, une cellule souche hématopoïétique, une cellule des voies aériennes, plus particulièrement une cellule trachéale ou pulmonaire, une cellule de l'épithélium respiratoire.

Plus largement, la présente invention concerne également un procédé pour introduire une substance active comportant des charges négatives à l'intérieur d'une cellule, caractérisé en ce que l'on met en contact des cellules cultivées sur un milieu approprié avec une suspension de complexes composé cationique/substance active comportant des charges négatives. Après un certain temps d'incubation les cellules sont lavées et récupérées. La vérification de l'introduction de la substance active peut être effectuée (éventuellement après lyse de la cellule) par tout moyen approprié.

Le procédé d'introduction est bien connu en soi. Par le terme «introduction» on entend que la substance active comportant des charges négatives est transférée dans la cellule et est localisée, en fin de procédé, à l'intérieur de ladite cellule ou au niveau de la membrane de celle-ci. Dans le cas où la substance active est un acide nucléique, on parlera plus particulièrement de «transfection». Dans ce cas, la vérification de la transfection de l'acide nucléique pourra être effectuée par tout moyen approprié, par exemple par mesure de l'expression du gène considéré ou de la concentration de la protéine exprimée.

L'invention concerne plus particulièrement l'utilisation d'un composé, d'une composition ou d'un complexe selon l'invention pour la préparation d'un médicament à but curatif, préventif ou vaccinal, destiné au traitement du corps humain ou animal, notamment par thérapie génique.

Selon une première possibilité, le médicament peut être administré directement *in vivo* (par exemple dans un muscle, dans les poumons par aérosol...). On peut également adopter l'approche *ex vivo* qui consiste à prélever des cellules du patient (cellules souches de la moëlle osseuse, lymphocytes du sang périphérique, cellules musculaires...), de les transfecter *in vitro* selon la présente invention et de les réadministrer au patient.

Les complexes selon l'invention peuvent être administrés par voie intramusculaire, intratrachéale, intranasale, intracérébrale, intrapleurale, intratumorale, intracardiaque, intragastrique, intrapéritonéale, épidermique, intraveineuse, intraartérielle par seringue ou tout autre moyen équivalent, les systèmes adaptés au traitement des voies aériennes ou des muqueuses tels que l'inhalation, l'instillation, ou l'aérosolisation. On peut également citer les modes d'administration par voie topique, tels que par exemple par application d'une crème, par administration orale ou tout autre moyen parfaitement connu de l'homme de l'art et applicable à la présente invention.

Il est également dans la portée de 1' invention de cibler des organes ou des tissus particuliers par administration, notamment par voie intraveineuse, d'un complexe selon l'invention préparé de façon à ajuster le rapport composé ou composition/substance thérapeutiquement active dans ledit complexe, la charge apparente du complexe (voir notamment Liu et al, 1997, Gene Therapy, 4, 517-523 ; Thierry et al., 1995, P.N.A.S., 92, 9742-9746).

L' invention concerne également un procédé de thérapie génique consistant à administrer à un patient une quantité appropriée d'une composition selon l'invention. Selon la présente invention et dans le cadre d'une thérapie génique in *vivo,* il est possible de répéter plusieurs fois, chez un patient donné, le procédé tel que proposé sans qu'aucune réaction immunitaire majeure ne soit déclenchée à l'encontre de l'un des composés administrés. L'administration peut avoir lieu en dose unique ou répétée une ou plusieurs fois après un certain intervalle de temps. L'administration répétée permettrait de réduire la quantité de substance thérapeutiquement active, d'ADN plus particulièrement, à administrer pour une dose donnée. La voie d'administration et le dosage appropriés varient en fonction de divers paramètres, par exemple de l'individu ou de la maladie à traiter ou encore du polynucléotide à transférer.

L'invention concerne plus particulièrement une préparation pharmaceutique comprenant au moins un complexe tel que décrit précédemment, renfermant éventuellement en outre au moins un adjuvant capable de stabiliser ladite préparation pharmaceutique en vue de son stockage par exemple et/ou d'améliorer le pouvoir de transfection dudit complexe. Un tel adjuvant pourrait par exemple être choisi parmi le groupe consistant en la chloroquine, un composé polaire protique notamment choisi parmi le propylène glycol, le polyéthylène glycol, le glycérol, l'éthanol, le 1-methyl L -2-pyrrolidone ou leurs dérivés, ou un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés. De même, ladite préparation peut renfermer un support acceptable d'un point de vue pharmaceutique permettant son administration à l'homme ou l'animal.

Dans le cadre de la mise en oeuvre d'un procédé de traitement *in vivo* selon la présente invention, il est en outre possible d'effectuer, avant l'administration d'une préparation pharmaceutique telle que décrite précédemment, un traitement du patient visant à observer une déplétion temporaire des macrophages permettant d'améliorer le taux de transfection. Une telle technique est décrite dans la littérature, voir notamment Van Rooijen et al., 1997, TibTech, 15, 178-184.

L'invention concerne une cellule transfectée par un complexe tel que défini précédemment, particulièrement une cellule procaryote, une cellule de levure ou eucaryote, notamment une cellule animale, en particulier une cellule de mammifère, et plus particulièrement une cellule cancéreuse. Selon un cas préféré de l'invention, ladite cellule est une cellule des voies aériennes, plus particulièrement une cellule trachéale ou pulmonaire, et avantageusement une cellule de l'épithélium respiratoire.

Enfin, L'invention concerne un dispositif ainsi qu'un procédé permettant l'isolement d'une molécules d'intérêt renfermant au moins une charge négative, notamment des acides nucléiques tels que définis selon la présente invention. Par «isolement», on entend désigner la séparation, la détection, l'enrichissement, la purification d'une fraction de molécules anioniques, selon une méthode d'isolement spécifique ou aspécifique, de manière qualitative et/ou quantitative.

Plus particulièrement, l'invention concerne undit dispositif consistant en un support solide dispersé, tel que par exemple des particules de polymères (de polystyrène, d'acrylamide, de méthacrylamide, ou de l'un de leurs dérivés ou tout autre polymère susceptible de former des particules dont de nombreux exemples sont décrits dans la littérature, notamment dans la littérature relative aux applications diagnostiques) ou en un support solide non dispersé tel que par exemple un tube, par exemple de polystyrène, une colonne, par exemple d'hydroxyapatite, une colonne en phase réverse ou équivalente, sur lequel est fixé au moins un composé ou une composition selon l'invention, sous sa forme cationique. La fixation audit support solide peut être réalisée de manière directe (adsorption par exemple) ou indirecte (par l'intermédiaire d'un pontage de type ligand /anti-ligand). La réalisation de tels dispositifs est à la portée de l'homme du métier.

Par ailleurs, l'invention concerne un procédé mettant en oeuvre un tel dispositif afin de permettre l'isolement de molécules anioniques, en particulier d'acides nucléiques. Un tel isolement peut notamment être non spécifique ou préférentiellement spécifique. Dans ce second cas, ledit composé ou ladite composition cationique est, préalablement à l'étape d' isolement, mis en contact avec, par exemple, un oligonucléotide dont la séquence spécifique permet, après une étape d'hybridation, dans des conditions permettant une hybridation spécifique, d'isoler de manière spécifique un fragment d'acide nucléique renfermant tout ou partie d'une séquence complémentaire à la séquence dudit oligonucléotide. La mise en oeuvre d'un tel procédé est largement décrite dans la littérature.

### LEGENDE DES FIGURES :

Figure 1 : Cette figure illustre le mode de synthèse des composés décrits en A.

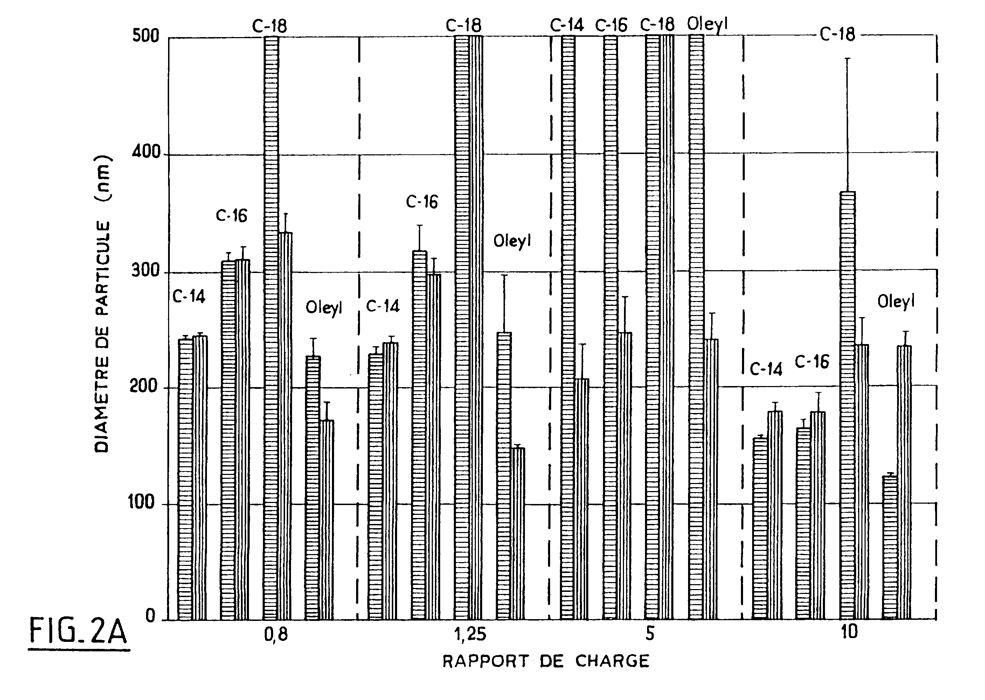

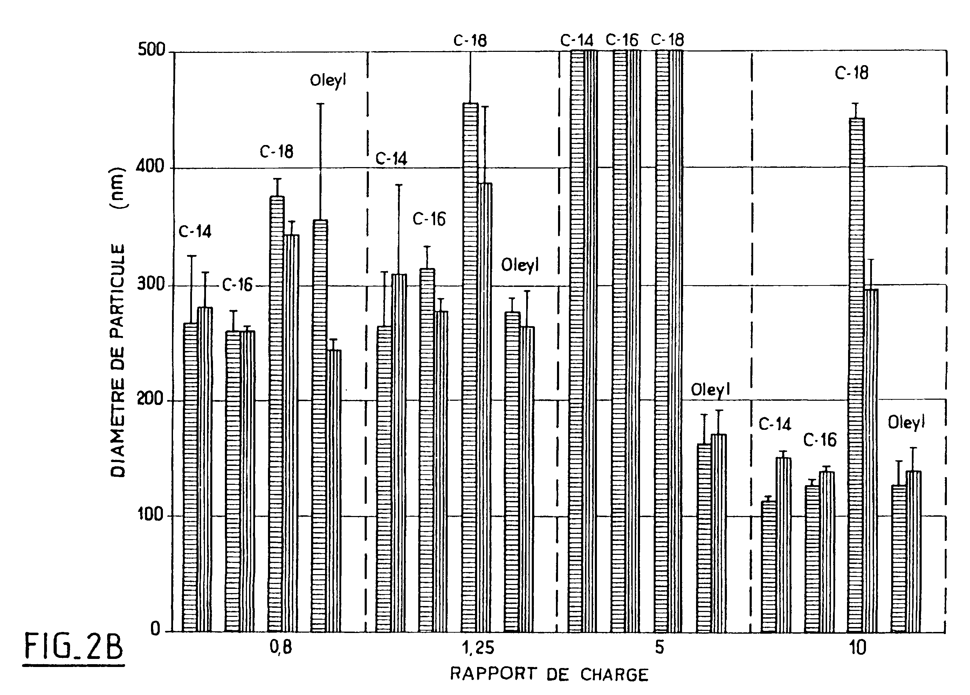

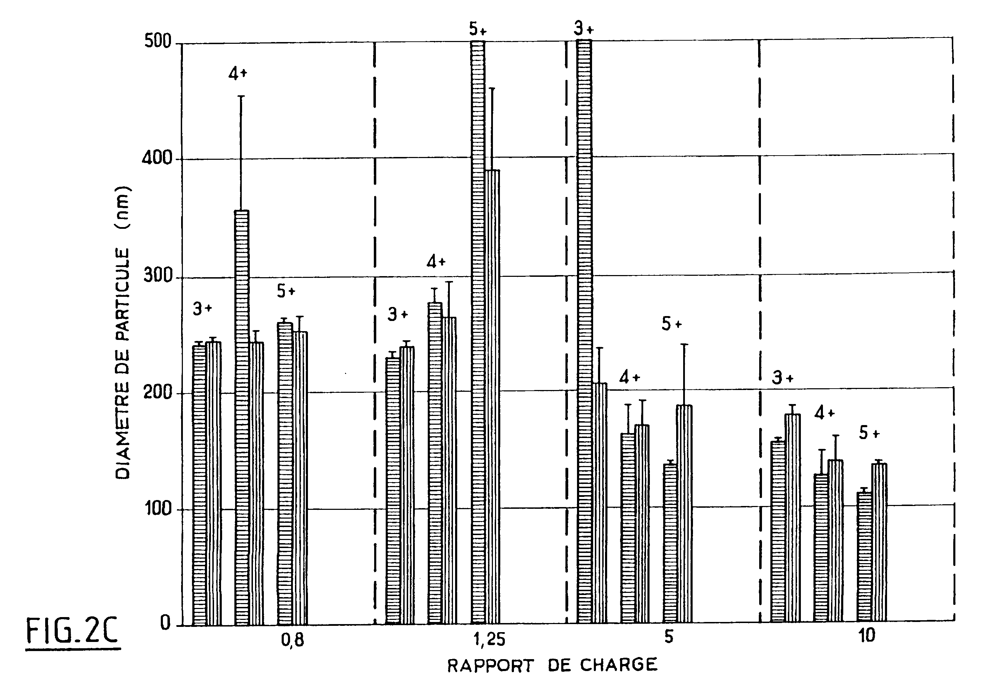

Figure 2 : Analyse de la taille des particules des complexes formés entre les glycérolipides de l'invention et le plasmide pTG11033 (patent application n°FR9708267) à 0.1 mg/ml préparer par la technique à l'éthanol décrite précédemment. Les résultats sont présentés pour différents rapports de charges. Pour chaque mesure, trois préparations indépendantes ont été testées et la mesure de la reproductibilité est évaluée par la déviation standard entre ces préparations. La taille des particules est mesurée par PCS (Photon Correlation Spectroscopy). Les colonnes hachurées figurent les complexes obtenus en l'absence de DOPE et les colonnes foncées en présence d'une quantité équimolaire de DOPE. Les complexes pour lesquels on observe une précipitation sont représentés par une colonne qui s'étend au delà de l'échelle. A) glycérolipides comportant 3 charges positives (C-14 = pcTG 18, C-16 = pcTG21, C-18 = pcTG19, oleoyl = pcTG20) ; B) glycérolipides comportant 4 charges positives (C-14 = pcTG 33, C-16 = pcTG34, C-18 = pcTG36, oleoyl = pcTG35) ; C) glycérolipides comportant des chaines oleoyl et 3 (pcTG 20), 4 (pcTG35), ou 5 charges positives (pcTG56).

Figure 3 : Injection intraveineuse de complexes selon l'invention. L'activité luciférase est indiquée en RLU/mg de protéine).

Figure 4 : Cette figure illustre le mode de synthèse des glycérolipides fluorés de l'exemple N.

Figure 5 A à G : Transfection in vitro de cellules A549 en présence de complexes renfermant différents glycérolipides fluorés selon l'invention.

### EXEMPLES :

Les exemples ci-après illustrent l'invention, sans la limiter en aucune façon, en liaison avec les figures annexées qui font partie intégrante de la description.

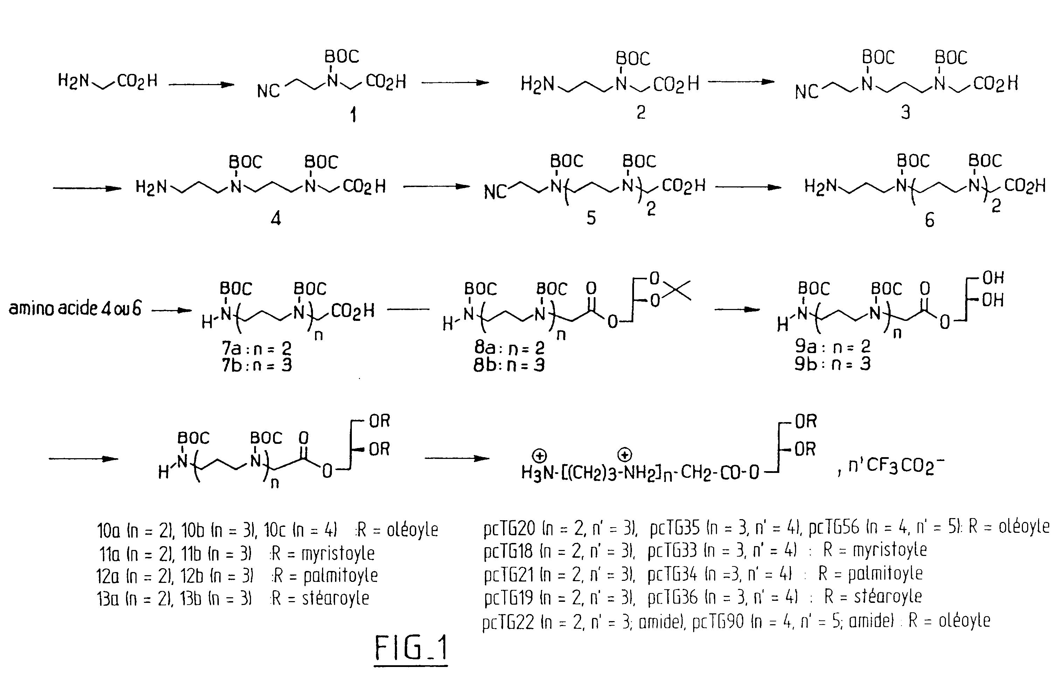

### A. :Synthèse des acides de formule VII avec m = 3 ; n = 2 R₆ = BOC (acide 7a) ou m = 3 ; n = 3 ; R₆ = BOC (acide 7b) (voir figure 1)

### Amino acides 4, 6 et 15

Une solution d'acrylonitrile (9,6 ml, 146 mmoles) dans 50 ml de 1,4-dioxane est ajoutée goutte à goutte à une solution glacée de glycine (10,0 g, 132 mmoles) et de soude 1N (133 ml) dans un mélange 1/1 d'eau et de 1,4-dioxane (200 ml). Le milieu réactionnel est agité à 0°C pendant 1 h et à température ambiante pendant 4 h supplémentaires. Une solution de dicarbonate de ditertiobutyle (35,0 g, 159 mmoles) dans le 1,4-dioxane (100 ml) est ensuite ajoutée goutte à goutte et le milieu réactionnel est agité pendant deux heures à température ambiante. Après extraction par l'éther (2 x 100 ml), la phase aqueuse est acidifiée (pH 2-3) avec de l'acide chlorhydrique 1N et extraite avec de l'acétate d'éthyle (2 x 100 ml). Les phases organiques combinées sont séchées ec concentrées. Le cyano acide obtenu 1 (24,4 g, Rdt 81%) se présente sous la forme d'un solide blanc de point de fusion 87-89°C.
RMN-¹H (200 MHz, D₂O) : δ 3,88 et 3,87 (2 s, 2 H, -CH₂-CO₂H), 3,48 et 3,45 (2 t, J = 6,3 Hz, 2 H, -CH₂-N(BOC)-), 2,58 et 2,56 (2 t, J = 6,3 et 6,4 Hz, 2 H, -CH₂-CN), 1,30 et 1,24 (2s, 9 H, t-Bu-).

Une solution d'acide 1 (11,5 g, 50,4 mmoles) dans 100 ml d'éthanol contenant 4,04 g (100 mmoles) de soude est hydrogénée en présence de nickel de Raney (3,2 g) pendant 18 h à température ambiante. Le mélange est filtré sur célite et le catalyseur lavé avec du méthanol (2 x 30 ml). Le filtrat est acidifié à pH 4-5 avec de l'acide chlorhydrique à 10 % et concentré sous vide pour donner un solide blanc qui est dissous dans le chloroforme (50 ml) pour précipiter la plus grande partie du chlorure de sodium. Après filtration, concentration sous vide du filtrat et recristallisation dans le tétrachlorure de carbone, on obtient l'amino acide 2 (10,4 g ; Rdt 89 %) dont le point de fusion est de 201-202°C.
RMN-¹H (200 MHz, D₂O) : δ 3,53 (s, 2 H, -CH₂-CO₂H), 3,17 (t, J = 6,6 Hz, 2 H, -CH₂-N(BOC)-), 2,83 (t, J =7,5 Hz, 2 H, -CH₂-NH₂), 1,69 (quint, J = 7 Hz, 2 H, -CH₂-), 1,26 et 1,21 (2s, 9 H, t-Bu-).
La même procédure que pour l'obtention du cyano acide 1 conduit à l'obtention du cyano acide 3 à partir de l'amino acide 2.
RMN-¹H (200 MHz, CDCl₃) : δ 4,00-3,85 (m, 2 H, -CH₂-CO₂H), 3,55-3,43 (m, 2 H, -CH₂-CH₂-CN), 3,31 (t, J = 7,2 Hz, 4 H, -CH₂-N(BOC)-), 2,61 (m, 2 H,-CH₂-CN), 1,78 (quint, J = 7,2 Hz, 2 H,-CH₂-), 1,47 et 1,44 (2s, 18 H, t-Bu-).

L'amino acide 4 est obtenu avec un rendement de 87 % après purification par chromatographie sur gel de silice (éluant : méthanol/dichlorométhane 3/7, puis 6/4) à partir du cyano acide 3 selon la même procédure qui a conduit à l'amino acide 2. Le point de fusion est 189-190°C.
RMN-¹H (200 MHz, D₂O) : δ 3,57 et 3,54 (2s, 2 H, -CH₂-CO₂H), 3,2-3,0 (m, 6 H, -CH₂-N(BOC)-), 2,80 (t, J = 7,7 Hz, 2 H, -CH₂-NH₂), 1,80-1,50 (m, 4 H, -CH₂-), 1,27 et 1,22 (2s, 18 H, t-Bu-).

La même procédure que pour le cyano acide 1 permet l'obtention du cyano acide 5 à partir de l'amino acide 4.
RMN-¹H (200 MHz, CDCl₃) : δ 3,85 (s large, 2 H, -CH₂-CO₂H), 3,47 (t, J = 6,6 Hz, 2 H, -CH₂-CH₂-CN), 3,35-3,05 (m, 8 H, -CH₂-N(BOC)-), 2,60 (m, 2 H, -CH₂-CN), 1,85-1,60 (m, 4 H, -CH₂-), 1,46 et 1,44 (2 s, 27 H, t-Bu-).

L'amino acide 6 est obtenu avec un rendement de 83 % après purification par chromatographie sur gel de silice (éluant : méthanol/dichlorométhane 3/7, puis 6/4) à partir du cyano acide 5 selon la même procédure qui a conduit à i'amino acide 2.
RMN-¹H (200 MHz, D₂O) : δ 3,76 et 3,73 (2s, 2 H, -CH₂-CO₂H), 3,25-2,75 (m, 12 H, -CH₂-N(BOC)- et -CH₂-NH₂), 1,85-1.50 (m, 6 H,-CH₂-), 1,28 et 1,23 (2s, 27 H,t-Bu-).
La même procédure que pour le cyano acide 1 permet l'obtention du cyano acide 14 à partir de l'amino acide 6.
RMN-¹H (200 MHz, CDCl₃):δ 3,95 et 3,87 (2 s larges, 2 H, -CH₂-CO₂H), 3,47 (t, J = 6,5 Hz, 2 H, -CH₂-CH₂-CN), 3,40-3,05 (m, 12 H, -CH₂-N(BOC)-), 2,61 (m, 2 H, -CH₂-CN), 1,90-1,60 (m, 6 H,-CH₂-), 1,47, 1,45 et 1,44 (3 s, 36 H, t-Bu-).

L'amino acide 15 est obtenu avec un rendement de 71% après purification par chromatographie sur gel de silice (éluant : méthanol/dichlorométhane 1/9 puis 3/7) à partir du cyano acide 14 selon une procédure identique à celle qui a conduit à l'amino acide 2.
RMN-¹H (200 MHz, D₂O - CD₃OD):δ 3,57 (m, 2 H, -CH₂-CO₂H), 3,15-2,80 (m, 14 H, -CH₂-N(BOC)-), 2,70 (t, J = 7,5 Hz, 2 H, -CH₂-NH₂), 1,75-1,35 (m, 8 H, -Ch₂-), 1,17 et 1,13 (2 s, 36 H, t-Bu-)

### Acides 7a, 7b et 7c

Une solution de dicarbonate de ditertiobutyle (1,09 g, 5,01 mmoles) dans le tétrahydrofurane (4 ml) est ajoutée à une solution d'amino acide 4 (1,50 g, 3,85 mmoles) et de triéthylamine (1,07 ml, 7,7 mmoles) dans un mélange 1/1 de tétrahydrofurane et d'eau (8 ml). Le milieu réactionnel est agité pendant 2 h à température ambiante. Il est alors acidifié à pH 3 par une solution aqueuse à 10% d'acide chlorhydrique et est extrait avec de l'acétate d'éthyle. La phase organique est lavée à l'eau, séchée sur sulfate de sodium et concentrée pour donner une huile incolore qui après chromatographie sur colonne de gel de silice (éluant : acétate d'éthyle) donne l'acide 7a. (1,79 g ; Rdt : 95%).
RMN-¹H (200 MHz, CD₃OD) : δ 3,79 (s, 2 H, -CH₂-CO₂H), 3,30-3,15 (m, 6 H, -CH₂-N(BOC)-), 3,03 (t, J = 6.7 Hz, 2 H, -CH₂-N(BOC)-), 1,85-1,60 (m, 4 H, -CH₂-), 1,46 et 1,43 (2 s, 27 H, t-Bu-)

La même procédure que pour l'acide 7a permet l'obtention de l'acide 7b (7,31 g ; Rdt : 95%) à partir de l'amino acide 6 (6,50 g).
RMN-¹H (200 MHz, CDCl₃) : δ 3,93 et 3,86 (m, 2 H, -CH₂-CO₂H), 3,40-3,05 (m, 12 H, -CH₂-N(BOC)-), 1,85-1,60 (m, 6 H, -CH₂-), 1.44 (s large, 36 H, t-Bu).

La même procédure que pour l'acide 7a permet l'obtention de l'acide 7c (3,37 g; Rdt : 92% après chromatographie sur gel de silice; éluant : méthanol/dichlorométhane 5/95 puis 10/90) à partir de l'amino acide 15 (3,20 g; 4,55 mmoles).
RMN-¹H (200 MHz, CDCl₃):δ 3,85 (m, 2 H, -CH₂-CO₂H), 3,45-3,05 (m, 16 H, -CH₂-N(BOC)-), 1,85-1,60 (m, 8 H, -CH₂-), 1,45, 1,44 et 1,43 (3 s, 45 H, t-Bu).

### Synthèse de glycérolipides cationiques

### Esters 8a, 8b et 8c

Une solution de dicyclohexylcarbodiimide (0,60 g, 2,9 mmoles) dans le dichlorométhane sec (1 ml) est ajoutée à une solution d'acide 7a (1,10 g, 2,25 mmoles), de (S)-(+)-2,2-diméthyl-1,3-dioxolane-4-méthanol (0,39 g, 2,9 mmoles) et de 4-(diméthylamino)pyridine (27 mg, 0,23 mmole) dans le dichlorométhane sec (3 ml). Le mélange réactionnel est agité pendant 18 h à température ambiante. Le précipité de dicyclohexylurée est enlevé par filtration et le filtrat est concentré sous vide puis chromatographié sur colonne de gel de silice (éluant: éther/hexane 6/4) pour àonner l'ester 8a (0,72 g; 53%).
RMN-¹H (200 MHz, CDCl₃) : δ 4,40-4,02 (m, 4 H, -CH₂-O-), 3,98 et 3,90 (2 s larges, 2 H, -CH₂-CO₂-), 3,73 (m, 1 H, CH-O-), 3,35-3,00 (m, 8 H), 1,85-1,55 (m, 4 H, -CH₂-), 1,45, 1,43 et 1,42 (3 s, 30 H), 1, 36 (s, 3 H, Me-).

La même procédure que pour l'ester 8a permet l'obtention de l'ester 8b (3,08 g; rdt : 49%) à partir de l'acide 7b (5,32 g, 8,22 mmoles).
RMN-¹H (200 MHz, CDCl₃) : δ 4,40-4,02 (m, 4 H, -CH₂-O-), 3,99 et 3,91 (2 s larges, 2 H, -CH₂C O₂-), 3,73 (m, 1 H, CH-O-), 3,32-3,00 (m, 12 H, -CH₂-N(BOC)-), 1,85-1,55 (m, 6 H, -CH₂-), 1,45, 1,44, 1,43 et 1,41 (4 s, 39 H, t-Bu- and Me-), 1,36 (2 s, 3 H, Me-).

La même procédure que pour l'ester 8a permet l'obtention de l'ester 8c (1,73 g; Rdt : 95%) à partir de l'acide 7c (1,60 g; 1,99 mmole).
RMN-¹H (200 MHz, CDCl₃):δ 4,35-4,04 (m, 4 H, -CH₂-O-), 3,99 et 3,92 (2 s, 2 H, -CH₂-CO₂-), 3,74 (m, 1 H, >CH-O-), 3,30-3,00 (m, 16 H, -CH₂-N(BOC)-), 1,85-1,55 (m, 8 H, -CH₂-), 1,46, 1,45, 1,44 et 1,42 (4 s, 48 H, t-Bu- et Me-), 1,36 (s, 3 H, Me-).

### Dihydroxyesters 9a, 9b et 9c

Une solution d'ester 8a (663 mg, 1,10 mmole) et d'acide chlorhydrique 1 N (0,44 ml) dans le méthanol (19 ml) est agitée pendant 16 h à température ambiante. De la triéthylamine (0,5 ml) est ensuite ajoutée jusqu'à neutralité. L'évaporation sous vide suivie de la chromatographie sur colonne de gel de silice (éluant : éther puis acétate d'éthyle) donne le dihydroxyester 9a (497 mg; rdt : 80%) sous la forme d'une huile incolore.
RMN-¹H (200 MHz, CDCl₃) : δ 4,26 (m, 2 H, -CH₂-OC(=O)-), 4,00-3,50 (m, 5 H, CH-OH, -CH₂OH et -CH₂-CO₂-), 3,40-3,00 (m, 8 H,-CH₂-N(BOC)-), 1,85-1,55 (m, 4 H, -CH₂-), 1,45 et 1,43 (2 s, 27 H, t-Bu-).

La même procédure que pour le dihydroxyester 9a permet l'obtention du dihydroxyester 9b (340 mg ; rdt : 71%) à partir de l'ester 8b (500 mg, 0,66 mmole).
RMN-¹H (200 MHz, CDCl₃) : δ 4,26 (m, 2 H, -CH₂-OC(=O)-), 4,00-3,40 (m, 5 H, CH-OH, -CH₂OH, et -CH₂-CO₂-), 3,40-3,00 (m, 12 H,-CH₂-N(BOC)-), 1,85-1,55 (m, 6 H, -CH₂-), 1,46, 1,45 et 1,43 (3 s, 36 H, t-Bu-).

La même procédure que pour le dihydroxyester 9a permet l'obtention du dihydroxyester 9c (1,23 g; Rdt : 83% après chromatographie sur gel de silice; éluant : méthanol/dichlorométhane 5/95) à partir de l'ester 8c (1,55 g; 1,69 mmole).
RMN-¹H (200 MHz, CDCl₃):δ 4,25 (m, 2 H, -CH₂-OC(=O)-), 4,00-3,40 (m, 5 H, CH-OH, -CH₂OH et -CH₂-CO₂-), 3,40-3,00 (m, 16 H, -CH₂-N(BOC)-), 1,90-1,60 (m, 8 H, -CH₂-), 1,46, 1,45, 1,44 et 1,42 (4 s, 45 H, t-Bu-).

### Triesters 10a, 11a, 12a et 13a

Une solution de dicyclohexylcarbodiimide (142 mg, 0,69 mmole) dans le dichlorométhane sec (1 ml) est ajoutée à une solution de dihydroxyester 9a (130 mg, 0,23 mmole), d'acide oléique (195 mg, 0,69 mmole ; Fluka puriss.) et de 4-(diméthylamino)pyridine (3 mg, 0,02 mmole) dans le dichlorométhane sec (2 ml). Le mélange réactionnel est agité pendant 16 h à température ambiante. Le précipité de dicyclohexylurée est enlevé par filtration et le filtrat est concentré sous vide et chromatographié sur colonne de gel de silice (éluant : éther/hexane 3/7 puis 4/6) pour donner le triester 10a (142 mg; 57%) sous forme d'une huile incolore.
RMN-¹H (200 MHz, CDCl₃) : δ 5,34 (m, 4 H, -CH=), 5,26 (m, 1 H, CH-OC(=O)-), 4,40-4,05 (m, 4 H, -CH₂-OC(=O)-), 3,95 et 3,88 (2 m, 2 H, -N(BOC)-CH₂-CO₂-), 3,35-3,00 (m, 8 H, -CH₂-N(BOC)-), 2,31 (t, J = 7,5 Hz, 4 H, -CH₂-CO₂-), 2,01 (m, 8 H, -CH₂-CH=), 1,85-1,50 (m, 8 H, -CH₂-), 1,46, 1,44 et 1,42 (3 s, 27 H, t-Bu-), 1,30 et 1,27 (2 s larges, 44 H, -CH₂-), 0,88 (t, J = 6,4 Hz, 6 H, Me-).

La même procédure que pour le triester 10a permet d'obtenir les triesters 11a (rdt : 65%) , 12a (rdt : 64%) et 13a (rdt : 58%) à partir du dihydroxyester 9a et respectivement de l'acide myristique, de l'acide palmitique et de l'acide stéarique.
11a RMN-¹H (200 MHz, CDCl₃) : δ 5,26 (m, 1 H CH-OC(=O)-), 4,45-4,05 (m, 4 H, -CH₂-OC(=O)-), 3,95 et 3,88 (2 m, 2 H, -N(BOC)-CH₂-CO₂-), 3,35-3,00 (m, 8 H, -CH₂-N(BOC)-), 2,31 (t, J = 7,5 Hz, 4 H, -CH₂-CO₂-), 1,85-1,50 (m, 8 H, -CH₂-), 1,46, 1,44 et 1,42 (3 s, 27 H, t-Bu-), 1,26 (s, 40 H, -CH₂-), 0,88 (t, J = 6.4 Hz, 6 H, Me-).
12a RMN-¹H (200 MHz, CDCl₃) : δ 5,26 (m, 1 H, CH-OC(=O)-), 4,40-4,05 (m, 4 H, -CH₂-OC(=O)-), 3,95 et 3,88 (2 m, 2 H, -N(BOC)-CH₂-CO₂-), 3,35-3,00 (m, 8 H, -CH₂-N(BOC)-), 2,31 (t, J = 7,5 Hz, 4 H, -CH₂-CO₂-), 1,85-1,50 (m, 8 H, -CH₂-), 1,46, 1,44 et 1,42 (3 s, 27 H, t-Bu-), 1,25 (s, 48 H, -CH₂-), 0,88 (t, J = 6,4 Hz, 6 H, Me-).
13a RMN-¹H (200 MHz, CDCl₃) : δ 5,26 (m, 1 H, CH-OC(=O)-), 4,40-4,05 (m, 4 H, -CH₂-OC(=O)-), 3,95 et 3,88 (2 m, 2 H, -N(BOC)-CH₂-CO₂-), 3,35-3,00 (m, 8 H, -CH₂-N(BOC)-), 2,31 (t, J = 7,5 Hz, 4 H, -CH₂-CO₂-), 1,85-1,50 (m, 8 H, -CH₂-), 1,46, 1,44 et 1,42 (3 s, 27 H, t-Bu-), 1,26 (s, 56 H, -CH₂-), 0,88 (t, J = 6,4 Hz, 6 H, Me-).

### Triesters 10b, 11b, 12b et 13b

La même procédure que pour le triester 10a permet l'obtention du triester 10b (137 mg ; rdt : 61%) à partir du dihydroxyester 9b (130 mg, 0,18 mmole) et d'acide oléique (153 mg, 0,54 mmole ; Fluka puriss.).
RMN-¹H (200 MHz, CDCl₃) : δ 5,34 (m, 4 H, -CH=) , 5,26 (m, 1 H, CH-OC(=O)-), 4,40-4,05 (m, 4 H, -CH₂-OC(=O)-), 3,96 et 3,89 (2 m, 2 H, -N(BOC)-CH₂-CO₂-), 3,35-3,00 (m, 12 H, -CH₂-N(BOC)-), 2,31 (t, J = 7,5 Hz, 4 H, -CH₂-CO₂-), 2,01 (m, 8 H, -CH₂-CH=), 1,85-1,50 (m, 10 H, -CH₂-), 1,46, 1,45, 1,44 et 1,41 (4 s, 36 H, t-Bu-), 1,30 et 1,27 (2 s larges, 44 H, -CH₂-), 0.88 (t, J = 6,4 Hz, 6 H, Me-).

La même procédure que pour le triester 10a permet d'obtenir les triesters 11b (rdt : 64%) , 12b (rdt : 583) et 13b (rdt : 63%) à partir du dihydroxyester 9b et respectivement de l'acide myristique, de l'acide palmitique et de l'acide stéarique.
11b RMN-¹H (200 MHz, CDCl₃) : δ 5,26 (m, 1 H, CH-OC(=O)-), 4,40-4,05 (m, 4 H, -CH₂-OC(=O)-), 3,97 et 3,89 (2 m, 2 H, -N(BOC)-CH₂-CO₂-), 3,35-3,00 (m, 12 H, -CH₂-N(BOC)-), 2,31 (t, J = 7,5 Hz, 4 H, -CH₂-CO₂-), 1,85-1,50 (m, 10 H, -CH₂-), 1,46, 1,45, 1,44 et 1,41 (4 s, 27 H, t-Bu-), 1,26 (s, 40 H, -CH₂-), 0,88 (t, J = 6,4 Hz, 6 H, Me-).
12b RMN-¹H (200 MHz, CDCl₃) : δ 5,26 (m, 1 H, CH-OC(=O)-), 4,40-4,05 (m, 4 H, -CH₂-OC(=O)-), 3,97 et 3,89 (2 m, 2 H, -N(BOC)-CH₂-CO₂-), 3,35-3,00 (m, 12 H, -CH₂-N(BOC)-), 2,31 (t, J = 7,5 Hz, 4 H, -CH₂-CO₂-), 1,85-1,50 (m, 10 H, -CH₂-), 1,46, 1,45, 1,44 et 1,41 (4 s, 27 H, t-Bu-), 1,25 (s, 48 H, -CH₂-), 0,88 (t, J = 6,4 Hz, 6 H, Me-).
13b RMM-¹H (200 MHz, CDCl₃) : δ 5,26 (m, 1 H, CH-OC(=O)-), 4,40-4,05 (m, 4 H, -CH₂-OC(=O)-), 3,97 et 3,39 (2 m, 2 H, -N(BOC)-CH₂-CO₂-), 3,35-3,00 (m, 12 H, -CH₂-N(BOC)-), 2,31 (t, J = 7,5 Hz, 4 H, -CH₂-CO₂-), 1,85-1,50 (m, 10 H, -CH₂-), 1,46,1,45, 1,44 et 1,41 (3 s, 27 H, t-Bu-), 1,25 (s, 56 H, -CH₂-), 0,88 (t, J = 6,4 Hz, 6 H, Me-).

### Triesters 10c

La même procédure que pour le triester 10a permet l'obtention du triester 10c (0,75 g; Rdt : 47%) à partir du dihydroxyester 9c (1,00 g; 1,14 mmole) et d'acide oléique (0,97 g; 3,42 mmoles; Fluta puriss.).
RMN-¹H (200 MHz, CDCl₃):δ 5,34 (m, 4 H, -CH=) , 5,26 (m, 1 H, CHOC(=O)-), 4,40-4,05 (m, 4 H, -CH₂-OC(=O)-), 3,95 et 3,89 (2 m, 2 H, -N(BOC)-CH₂-CO₂-), 3,35-3,00 (m, 16 H, -CH₂-N(BOC)-), 2,31 (t, J = 7,5 Hz, 4 H, -CH₂-CO₂-), 2,01 (m, 8 H, -CH₂-CH=), 1,85-1,50 (m, 12 H, -CH₂-), 1,46, 1,44, 1,43 et 1,41 (4 s, 45 H, t-Bu-), 1,30 et 1,27 (2 s large, 44 H, -CH₂-), 0,88 (t, J = 6,4 Hz, 6 H, Me-).

### Glycérolipides pcTG20

Le triester 10a (120 mg, 0,11 mmole) en solution dans le dichlorométhane sec (1 ml) est traité pendant 3 h avec un mélange 1/1 d'acide trifluoracétique et de dichlorométhane sec (20 ml) à 0°C. On ajoute ensuite de l'hexane (50 ml) et le mélange est évaporé sous vide et donne un film qui est mis en suspension (vortex) dans de l'éther distillé (5 à 10 ml). La filtration donne une poudre blanche qui est lavée avec de l'éther et séchée sous vide pour donner le glycérolipide pcTG20 (124 mg; 99%). Point de fusion : se décompose à 160°C.
RMN-¹H (200 MHz, CDCl₃ - CF₃C O₂D) : δ 5,34 (m, 5 H, -CH= et CHOC(=O)-), 4,60 - 4,15 (m, 4 H, -CH₂-OC(=O)-), 3,99 (s large, 2 H, -NH₂⁺-CH₂-CO₂-), 3,45-3,20 (m, 8 H, -CH₂-NH₂⁺-), 2,39 (t, J = 7,5 Hz, 4 H, -CH₂-CO₂-), 2,50-2,20 (m, 4 H, -CH₂-CH₂-NH₂⁺-), 2,01 (m, 8 H, -CH₂-CH=), 1,61 (m, 4 H, -CH₂-CH₂-CO₂-) , 1,30 et 1,27 (2 s, 44 H, -CH₂-), 0,88 (t, J = 6,4 Hz, 6 H, Me-).

Analyse élémentaire : Calculé pour C₅₃H₉₂F₉N₃O₁₂ : C, 56,12%; H, 8,18% ; N, 3,70%. Trouvé : C, 56,3% ; H, 7,9% ; N, 3,7%.

### Glycérolipide pcTG35

La même procédure que pour le lipide cationique pcTG20 permet l'obtention du lipide cationique pcTG35 (101 mg; rdt : 97%) à partir du triester 10b (100 mg, 0,08 rnmoie). Point de fusion : se décompose à 190°C. RMN-¹H (200 MHz, CDCl₃ - CF₃C O₂D) : δ 5,35 (m, 5 H, -CH= et CH-OC(=O)-), 4,60-4,15 (m, 4 H, -CH₂-OC(=O)-), 4,00 (s large, 2 H, -NH₂⁺-CH₂-CO₂-), 3,45-3,10 (m, 12 H, -CH₂-NH₂⁺-), 2,40 (t, J = 7,5 Hz, 4 H, -CH₂-CO₂-), 2,45-2,20 (m, 6 H, -CH₂-CH₂-NH₂⁺-), 2,01 (m, 8 H, -CH₂-CH=), 1,60 (m, 4 H,-CH₂-CH₂-CO₂-), 1,30 et 1,27 (2 s, 44 H, -CH₂-), 0,87 (t, J = 6,4 Hz, 6 H, Me-).

Analyse élémentaire : Calculé pour C₅₈H₁₀₀F₁₂N₄O₁₄ : C, 53,36%; H, 7,72%;
N, 4,29%. Trouvé : C, 53,2% ; H, 7,6% ; N, 4,3%. La spectrométrie de masse a été mesurée à 849,8 Da (calculée : 849,3 Da).

### Glycérolipide pcTG56

La même procédure que pour le glycérolipide pcTG20 permet l'obtention du glycérolipide pcTG56 (0,51 g; Rdt : 93%) à partir du triester 10c (0,52 g; 0,37 mmole). Point de fusion : se décompose à 220°C.
RMN-¹H (200 MHz, CDCl₃ - CF₃CO₂D):δ 5,36 (m, 5 H, -CH= et CHOC(=O)-), 4,60-4,15 (m, 4 H, -CH₂-OC(=O)-), 4,00 (s large, 2 H, -NH₂⁺-CH₂-CO₂-), 3,45-3,10 (m, 16 H, -CH₂-NH₂⁺-), 2,41 (t, J = 7,5 Hz, 4 H, -CH₂-CO₂-), 2,28 (m, 8 H, -CH₂-CH₂-NH₂⁺-), 2,01 (m, 8 H, -CH₂-CH=), 1,61 (m, 4 H, -CH₂-CH₂-CO₂-), 1,30 et 1,27 (2 s, 44 H, -CH₂-), 0,87 (t, J = 6,4 Hz, 6 H, Me-).

### Glycérolipides pcTG18, pcTG21 et pcTG19

La même procédure que pour le glycérolipide pcTG20 permet d'obtenir les glycérolipides pcTG18 (rdt : 97%; solide; se décompose à 165°C), pcTG21 (rdt : 96%; solide; se décompose à 170°C) et pcTG19 (rdt : 92% ; solide ; se décompose à 186°C) à partir respectivement des triesters 11a, 12a et 13a.
pcTG18 RMN-¹H (200 MHz, CDCl₃-CF₃ C O₂D) : δ 5,35 (m, 1 H, CHOC(=O)-), 4,60-4,15 (m, 4 H, -CH₂-OC(=O)-), 3,97 (s large, 2 H, -NH₂⁺-CH₂-CO₂-), 3,45-3,15 (m, 8 H, -CH₂-NH₂⁺-), 2,38 (t, J = 7,5 Hz, 4 H, -CH₂-CO₂-) , 2,45-2,20 (m, 4 H, CH₂-CH₂-NH₂⁺-), 1,60 (m, 4 H, -CH₂-CH₂-CO₂-), 1,25 (s, 40 H, -CH₂-), 0,87 (t, J = 6,4 Hz, 6 H, Me-). Spectrométrie de masse : calculée 684,1 Da ; mesurée 684,5 Da.
pcTG21 RMN-¹H (200 MHz, CDCl₃-CD₃OD) : δ 5,20 (m, 1 H, CHOC(=O)), 4,45-4,00 (m, 4 H, -CH₂-OC(=O)-), 3,78 (s large, 2 H, -NH₂⁺-CH₂-CO₂-), 3,15-2,40 (m, 8 H, -CH₂-NH₂⁺-), 2,24 (t, J = 7,5 Hz, 4 H, -CH₂-CO₂-), 2,18-1,92 (m, 4 H, -CH₂-CH₂-NH₂⁺-), 1,52 (m, 4 H, -CH₂-CH₂-CO₂-), 1,17 (s, 48 H, -CH₂-), 0,79 (t, J = 6,4 Hz, 6 H, Me-). Spectrométrie de masse : calculée : 740,2 Da ; mesurée 740,6 Da.
pcTG19 RMN-¹H (200 MHz, CDCl₃-CF₃ C O₂D) : δ 5,38 (m, 1 H, CHOC(=O)-), 4,60-4,15 (m, 4 H, -CH₂-OC(=O)-), 4,01 (s large, 2 H, -NH₂⁺-CH₂-CO₂-), 3,45-3,20 (m, 8 H, -CH₂-NH₂⁺-), 2,41 (t, J = 7,5 Hz, 4 H, -CH₂-CO₂-), 2,50-2,25 (m, 4 H, -CH₂-CH₂-NH₂⁺-), 1,61 (m, 4 H, -CH₂-CH₂-CO₂-), 1,26 (s, 56 H, -CH₂-), 0,88 (t, J = 6,4 Hz, 6 H, Me-). Spectrométrie de masse : calculée 796,3 Da ; mesurée 796,8 Da.

### Glycérolipides pcTG33, pcTG34 et pcTG36

La même procédure que pour le glycérolipide pcTG20 permet d'obtenir les glycérolipides pcTG33 (rdt : 97% ; solide ; se décompose à 205 °C), pcTG34 (rdt : 94% ; solide ; se décompose à 215 °C) et pcTG36 (rdt : 92% ; solide ; se décompose à 220 °C) à partir respectivement des triesters llb, 12b et 13b.
pcTG33 RMN-¹H (200 MHz, CDCl₃-CF₃ C O₂D) : δ 5,37 (m, 1 H, CHOC(=O)-), 4,60-4,15 (m, 4 H, -CH₂-OC(=O)-), 4,00 (s large, 2 H, -NH₂+-CH₂-CO₂-), 3,45-3,15 (m, 12 H, -CH₂-NH₂⁺-), 2,39 (t, J = 7,5 Hz, 4 H, -CH₂-CO₂-), 2,45-2,17 (m, 6 H, -CH₂-CH₂-NH₂⁺-), 1,60 (m, 4 H, -CH₂-CH₂-CO₂-), 1,25 (s, 40 H, -CH₂-), 0,87 (t, J = 6,4 Hz, 6 H, Me-). Spectrométrie de masse : calculée 741,2 Da ; mesurée 741,6 Da
pcTG34 RMN-¹H (200 MHz, CDCl₃-CF₃ C O₂D) : δ_5,37 (m, 1 H, CHOC(=O)-), 4,60-4,15 (m, 4 H, -CH₂-OC(=O)-), 3,98 (s large, 2 H, -NH₂⁺-CH₂-CO₂-), 3,45-3,10 (m, 12 H, -CH₂-NH₂⁺-), 2,39 (t, J = 7,5 Hz, 4 H, -CH₂-CO₂-), 2,45-2,15 (m, 6 H, CH₂-CH₂-NH₂⁺-) , 1,60 (m, 4 H, -CH₂-CH₂-CO₂-), 1,25 (s, 48 H, -CH₂-), 0,87 (t, J = 6,4 Hz, 6 H, Me-). Spectrométrie de masse : calculée 797,3 Da ; mesurée 797,8 Da.
pcTG36 RMN-¹H (200 MHz, CDCl₃-CF₃ C O₂D) : δ 5,35 (m, 1 H, CHOC(=O)-), 4,60-4,15 (m, 4 H, -CH₂-OC(=O)-), 3,98 (s large, 2 H, -NH₂⁺-CH₂-CO₂-) , 3,45-3,10 (m, 12 H, -CH₂-NH₂⁺-), 2.37 (t, J = 7,5 Hz, 4 H, -CH₂-CO₂-), 2,45-2,15 (m, 6 H, *-*CH₂-CH₂-NH₂⁺-), 1,59 (m, 4 H, -CH₂-CH₂-CO₂-) , 1,25 (s, 56 H, -CH₂-), 0,87 (t, J = 6,4 Hz, 6 H, Me-). Spectrométrie de masse : calculée 854,4 Da ; mesurée 854,0 Da.

### Glycérolipide pcTG22

Une voie un peu différente de celle décrite ci-dessus a permis l'obtention du glycérolipide pcTG22 à partir du 3-amino-1,2-propanediol.
RMN-¹H (200 MHz, CDCl₃ - CD₃OD) : δ 5,04 (m, 1 H, CH-OC(=O)-), 4,27-3,92 (m, 2 H, -CH₂-OC(=O)-), 3,63 (s large, 2 H, -CH₂-C(=O)-NH-), 3,10-2,90 (m, 8 H, -CH₂-NH₂⁺-), 2,24 et 2,23 (2 t, J = 7,5 Hz, 4 H, -CH₂-CO₂-), 2,15-1,95 (m, 4 H, -CH₂-CH₂-NH₂⁺), 1,51 (m, 4 H, -CH₂-CH₂-CO₂-), 1.17 (s, 56 H, -CH₂-), 0,79 (t, J = 6,7 Hz, 6 H, Me-). Spectrométrie de masse : calculée 795,3 Da ; mesurée 795,3 Da.

### Glycérolipide pcTG90

Une procédure analogue à celle décrite pour la synthèse du lipide pcTG56 permet l'obtention du lipide pcTG90 en utilisant le 3-amino-1,2 propanediol à la place du glycérol.

### B : Préparation des complexes glycérolipides-ADN par mise en solution dans l'éthanol

### 1. Préparation des complexes glycérolipides pcTG20, éventuellement DOPE,-ADN

Les quantités de lipides sont calculées en se basant sur la concentration d'ADN final (0,1 mg/ml pour les essais en cultures cellulaires), le rapport de charges désiré, la masse molaire et le nombre de charges positives du lipide cationique choisi. Pour obtenir un complexe entre pcTG20/DOPE et de l'ADN plasmidique à un rapport de 10 entre charges positives apportées par le lipide cationique et charges négatives apportées par l'ADN à une concentration d'ADN finale de 0,1 mg/ml, on mélange les différents ingrédients selon le calcul suivant :
0,1 mg d'ADN/ml soit (0,1/330) mmoles de charges négatives (330Da est le poids moléculaire moyen d'un nucléotide) par ml correspondent à : 0,30 µmole/ml de charges négatives. Pour obtenir 10 fois plus de charges positives il faut une concentration de 3,0 µmole/ml de charges positives apportées par le lipide cationique. La masse molaire de pcTG20 sous forme de trifluoroacétate est de 1134 g/mole et la molécule contient 3 charges positives. Donc il faut 1,0 µmole/ml de pcTG20 ce qui correspond à 1,13 mg/ml.

Pour obtenir une concentration équimolaire en L-α-Dioleoyl-phosphatidyléthanolamine (DOPE, 744 g/mole, Sigma ; P0510) il en faut 0,74 mg/ml dans la préparation lipidique. Les quantités et les concentrations pour les autres composés sont ajustées en conséquence de leurs masses molaires respectives et de leur nombre de charges positives.

Les lipides repris dans du chloroforme sont évaporés puis solubilisés dans du chloroforme:méthanol (v:v) et à nouveau évaporés. Les lipides cationiques sont pesés et la quantité de DOPE est ajoutée à partir d'une solution mère de 10 ou 20 mg/ml dans du chloroforme dans un tube en verre stérilisé à l'alcool et aux UV pour obtenir une concentration de 2mM en lipide cationique. Les solvants sont évaporés sous vide (200 mbar) pendant 45 min à 45°C en utilisant un vortex de 40 tours par minute (Labconco, Rapidvap, Uniequip, Martinsried, Allemagne). Le film lipidique est repris dans de l'éthanol pour être à la concentration de 50 mg/ml en lipide cationique.
pcTG20/DOPE 1,13 mg + 0,74 mg = 1,87 mg dans 23 µl d'ethanol. Cette solution est complétée à 230 µl par 20mM HEPES pH 7,5 pour préparer une solution à 5mg/ml finale en lipide cationique.

L'ADN plasmidique est préparé dans un tube plastique à partir d'une solution mère à 1 mg/ml (Tris 10mM, EDTA 1mM, pH 7,5).

Pour une solution de 0,5 ml finale, on prélève 50 µl de la solution mère (50 µg ADN) auquel on ajoute 335 µl de 20mM HEPES pH 7,5. Pour complexer l'ADN avec des préparations lipidiques, on ajoute les lipides sur l'ADN. La suspension est mélangée par aspiration/refoulage à la pipette (10-fois). Les complexes sont conservés à + 4°C.

115 µl de pcTG20/DOPE sont ajoutés aux 385 µl de la solution ADN pour obtenir 0,5 ml de complexe à 0,1 mg/ml ADN et à un rapport de charges de 10.

La préparation des complexes se fait sous une hotte à flux laminaire.

On obtient les complexes dont les caractéristiques sont indiquées dans le tableau I ci-après.

### 2. Préparation des complexes glycérolipides pcT35, pcTG22 et pcTG18 éventuellement DOPE,-ADN

Selon le même protocole on obtient les complexes dont les caractéristiques sont indiquées dans le tableau I ci-après.

### C . Préparation des complexes glycérolipides-ADN par mise en suspension dans une solution de détergent

### 1. Préparation des complexes glycérolipides pcTG20, éventuellement DOPE,-ADN

Les quantités de lipides sont calculées comme décrit ci-dessus en se basant sur la concentration d'ADN final (0,1 mg/ml pour les essais *in vitro),* le rapport de charges désiré, la masse molaire et le nombre de charges positives du lipide cationique choisi. Le mélange des lipides se fait dans un tube en verre, stérilisé à l'alcool et aux UV, pour obtenir une solution 2mM en lipide cationique (voir ci dessus). Les solvants sont évaporés et le film lipidique est repris dans une solution de n-octyl, β-D-glucopyranoside (octylglucoside, Sigma, O 9882) selon un rapport lipide cationique/détergent de 1:5 (mole : mole).

On prélève 253 µl d'une solution d'octylglucoside 20 mM dans de l'HEPES 20 mM pH 7,5 avec lesquels on reprend le film de mélange lipidique pcTG20/DOPE. L'ADN plasmidique est préparé à partir d'une solution mère d'ADN plasmidique à 1 mg/ml dont 50 µl sont placés dans 0,5 ml (0,1 mg/ml final) auquel on ajoute 323,5 ul d'HEPES 20 mM pH 7,5. 126,5 µl de la suspension lipidique sont ajoutés sur l'ADN en aspirant et refoulant 10 fois à la pipette pour obtenir la suspension finale à 0,1 mg/ml d'ADN et un rapport de charges +/- de 10. Pour éliminer le détergent, une dialyse de 3 fois 4 heures à température ambiante contre de l'HEPES 20 mM pH 7,5 est entreprise dans des microdoigts de dialyse (seuil d'exclusion de 13,2 kD ; Sartorius, Gôttingen, Allemagne). Les complexes ADN/lipide dialysés sont conservés à + 4°C. La préparation se fait dans une hotte à flux laminaire.

On obtient les complexes dont les caractéristiques sont indiquées dans le tableau I ci-dessous.

### 2. Préparation des complexes glycérolipides pcTG35, éventuellement DOPE,- ADN

On applique le même protocole que précédemment.

### D. Préparation des complexes lipides-ADN par sonication extrusion

Les quantités de lipides sont calculées comme décrit ci-dessus en se basant sur la concentration d'ADN final (0,1 mg/ml pour les essais *in vitro),* le rapport de charges désiré, la masse molaire et le nombre de charges positives du lipide cationique choisi. Le mélange des lipides se fait dans un tube en verre, stérilisé à l'alcool et aux UV, pour obtenir une solution 2mM en lipide cationique, comme indiqué ci-dessus. Les solvants sont évaporés et le film lipidique est repris dans 900 µl d'HEPES 20 mM pH 7,5 à 4°C pendant environ 16h. La suspension est soniquée dans un bain de sonication (Bransonic 221) jusqu'à homogéneité visuelle. La suspension lipidique est extrudée au travers de deux membranes de 0,2 µm de diamètre de pores (Nucleopore, Costar, Cambridge, MA, USA) et rinçée avec de l'HEPES 20 mM pH 7,5 (Extruder de Lipex Biomembranes, Vancouver, Canada) à une pression maximale de 50 bars. La suspension lipidique est maintenue à température ambiante pendant 1 heure. 450 µl de la suspension lipidique sont ajoutés sur 50 µl d'une solution mère de l'ADN plasmidique (1 mg/ml) et mélangés en aspirant/refoulant 10 fois à la pipette. Les complexes lipide/ADN sont conservés à +4°C. Les préparations sont faites sous une hotte à flux laminaire.

### E . Protocole d'évaluation de la complexation de l'ADN par les lipides

Un gel à 1% (w:v) d'agarose est préparé dans un tampon TAE (TAE : Tris 4,8 g/l + acétate de sodium 0,68 g/1 + EDTA 0,336 g/i pH 7,8). Si nécessaire, l'échantillon est dilué dans du TAE puis on ajoute le tampon d'échantillon (0,083% bleu de bromophénol, 0,083% cyanol xylène FF, 10% glycérol dans l'eau) de façon à se trouver à 50 ngADN/µl. L'échantillon est brièvement homogénéisé au vortex et laissé 30 min à température ambiante. A titre de témoin, on utilise le plasmide non-complexé préparé à la même concentration. 10 µl (500 ng d'ADN) sont déposés sur le gel et la migration s'effectue à 60mV pendant 3 heures. Le gel est révélé dans du TAE contenant 0,006% (v:v) de bromure d'éthidium à 10 mg/ml pendant au moins 30 min. Ensuite le gel est rincé dans du TAE et analysé sous UV.

### F . Protocole de mesure de la taille des particules par diffusion quasi élastique de la lumière

Les analyses sont faites sur un Coulter N4Plus (Coultronics France S.A., Margency, France) à 25°C après équilibration de l'échantillon pendant 20 min. Une aliquote de l'échantillon est aspirée et refoulée plusieurs fois avant d'être pipettée. L'échantillon est dilué dans la cuve de mesure et homogénisé. La mesure de la lumière diffractée à 90° est faite pendant 180 sec après 180 sec d'attente. La gamme utilisée va de 3 nm à 10 000 nm en utilisant 31 bins. Pour être valable, l'échantillon doit donner entre 50 000 et 1 000 000 counts/sec.

### G . Caractéristiques physico-chimiques

Les trois méthodes de formulation "injection d'éthanol", "dialyse de détergent" et «sonication/extrusion» sont appliquées aux lipides cationiques selon l'invention avec ou sans des quantités équimolaires de DOPE à des rapports de charges d'environ 10, ou 5. Les formulations sont considérées comme appropriées lorsque l'ADN est complètement complexé (aucune migration dans le gel d'agarose) et que les complexes présentent un diamètre déterminé par diffusion quasi élastique de la lumière inférieur à 500 nm. Le tableau résume les résultats de ces analyses. Tous les complexes ADN/lipides indiqués dans le tableau complexent l'ADN complètement aux rapports de charges analysés.

**TABLEAU I**

| glycérolipide cationique | Rapport¹ | Taille (nm)² | Formulation |
|---|---|---|---|
| pcTG20/DOPE | 10 | 84 +/-20 | éthanol |
| pcTG20/DOPE | 10 | 212 +/-141 | détergent |
| pcTG20/DOPE | 5 | 96 +/-17 | éthanol |
| pcTG35/DOPE | 10 | 67 +/-22 | éthanol |
| pcTG35/DOPE | 5 | 93 +/-39 | éthanol |
| pcTG35 | 10 | 83 +/-11 | éthanol |
| pcTG35 | 5 | 69³ | éthanol |
| pcTG33 | 10 | 223 +/-138 | sonication |
| pcTG33/DOPE | 10 | 243 +/-172 | sonication |
| pcTG22 | 10 | 79 +/-59 | éthanol |
| pcTG22 | 5 | 87 +/-52 | éthanol |
| pcTG22/DOPE | 10 | 261 +/-98 | sonication |
| pcTG22/DOPE | 10 | 89 +/-146 | éthanol |
| pcTG22/DOPE | 5 | 72 +/-44 | éthanol |
| pcTG18 | 10 | 68 +/-53 | éthanol |
| pcTG18 | 5 | 72 +/-30 | éthanol |
| pcTG18/DOPE | 10 | 62 +/-43 | éthanol |
| pcTG18/DOPE | 5 | 109 +/-64 | éthanol |

| | | | |
|---|---|---|---|
| 1:Rapport entre les charges positives du lipide cationique et les charges négatives de l'ADN. | | | |
| 2: déterminée 24 à 48 heures après la préparation (+/- représente la déviation standard de la mesure). | | | |
| 3 : déterminée 8 jours après la préparation | | | |

Ces analyses montrent que les formulations remplissent les exigences requises. La méthode "injection d'éthanol" donne les meilleurs résultats pour de nombreuses préparations qui présentent une taille inférieure à 100 nm. Les méthodes par dialyse de détergent et sonication/détergent permettent également d'obtenir des complexes répondant aux objectifs de la présente invention.

### H . Transfection in vitro de cellules satellites

Des cultures de cellules de muscles de chien et de muscles humains sont effectuées dans un milieu HamF 14 (Life Technologies) supplémenté avec 10 % de sérum de veau foetal (SVF, Hyclone, Logan, UT), 10 µg/ml d'insuline (Sigma), 10 ng/ml d'EGF (Sigma) et de FGF (Pepro Teck Inc, Rocky Hill, NJ) 2mM de glutamine (bioMérieux), et 40 µg/ml de gentamycine (Schering Plough).

Les cellules sont ensemencées 24 h à 48 h avant la transfection, dans une boîte de culture à 96 puits avec environ 5x10³ à 10⁴ cellules par puits, à environ 30 % de confluence, et maintenues à 37°C en atmosphère 5 % CO₂ et 95 % d'air.

Les transfections sont effectuées avec des mélanges de quantités variables de lipides et d'ADN plasmidique pour déterminer les rapports de charges et les concentrations d'ADN optimales par puits.

Les complexes utilisés sont préparés 24 h à 48 h avant la transfection et dilués dans HamF 14 plus 40 µg/ml de gentamycine et de la glutamine 2mM.

Après élimination du milieu de culture, 100 µl de mélanges de transfection avec ou sans 10 % de FCS sont transférés dans chacun des puits et les plaques sont incubées pendant 4 h à 37°C.

Tous les milieux de transfection sont alors ajustés à 10 % de SVF, 10 µg/ml d'insuline (Sigma), 10 ng/ml d'EGF (Sigma) et de FGF (Pepro Tech Inc, Rocky Hill, NJ), de la glutamine 2mM (bioMérieux), et 40 µg/ml de gentamycine (Schering Plough) pour un volume final de 250 µl. Les cultures sont incubées pendant 48 h puis les cellules sont récupérées et testées pour leur capacité à exprimer le gène de la luciférase. Les concentrations de protéines sont déterminées par le système de test de quantité de protéine (Promega).

### I . Transfection de cellules A549 avec des complexes lipidiques

Les cellules A549 (cellules épithéliales dérivées de carcinome pulmonaire humain) sont mises en culture dans du milieu d'Eagle modifié par Dulbecco (DMEM) contenant 10% de sérum de veau foetal (Gibco BRL) 24 heures avant le début de la transfection dans des plaques de 96 puits (2 x 10⁴ cellules par puits) dans une atmosphère humide à 37°C et 5% CO₂/95% air. Pour la transfection en l'absence de sérum, le milieu est enlevé et remplacé par du milieu sans sérum. Dans une autre microplaque les suspensions de complexes lipides/ADN suivantes sont préparées (complexes lipides/ADN à 0,1 mg/ml d'ADN et au rapport de charges indiqué) : 44 µl (4,4 µg ADN), 22 µl (2,2 µg ADN), 5,5 µl (0,55 µg ADN) de solution stock dans les 3 premiers puits, et 11 µl 10,11 µg ADN) de la solution stock diluée 10 fois dans le puits suivant. Le volume est complété à 110 µl avec du DMEM et 100 µl sont transférés sur les cellules A549. L'incubation se fait avec 4, 2, 0,5 et 0,1 µg d'ADN par puits pendant 4 heures. Ensuite, 50 µl de DMEM + 30% de sérum de veau foetal sont ajoutés 4 heures après le début de la transfection puis 100 µl de DMEM + 10% FCS 24 heures après le début de la transfection. Les transfections en présence de 10% de sérum de veau foetal sont réalisées d'une façon identique sauf que la transfection se passe dans du milieu avec sérum.

### J. Analyse de la transfection

48 h après la transfection, le milieu est éliminé et les cellules sont lavées avec 100 µl de solution phosphate PBS et lysées avec 50 µl de tampon de lyse (Promega) . Les lysats sont congelés à -80 °C jusqu'à la mesure de l'activité luciférase exprimée. Celle-ci se fait sur 20 µl de mélange pendant une minute en utilisant le kit de détermination de la "Luciférase» (Promega) (luminomètre LB96P Berthold) dans des plaques à 96 puits en mode cinétique.

### K .Transfection in vitro

Quelques-unes de ces préparations ont été évaluées en transfection *in vitro* en utilisant les cellules A549 et les cellules satellites primaires de chien.

Les résultats sont résumés dans le tableau II ci-dessous et montrent les unités relatives de lumière (RLU) par puits. Les valeurs données sont obtenues avec 2 µg d'ADN par puits. Tous les complexes sont préparés en utilisant la méthode d'injection d'éthanol. La concentration totale de protéine par puits est déterminée par les techniques conventionnelles (test 3CA, Pierce). A titre indicatif, un puits contient environ 20 à 30 µg de protéine.

**TABLEAU II**

| Lipide | rapport ¹ | A549² | A549+ sérum | myoblastes | myoblastes + sérum |
|---|---|---|---|---|---|
| pcTG20/DOPE | 10 | 3,6 x 10⁵ | 6,1 x 10⁶ | 8,9 x 10⁵ | 2,3 x 10⁷ |
| pcTG20/DOPE | 5 | 8,25 x 10⁶ | 2,8 x 10⁷ | 8,8 x 10⁵ | 1,3 x 10⁶ |
| pcTG35/DOPE | 10 | 4 x 10⁶ | 1,0 x 10⁷ | 7,1 x 10³ | 6,5 x 10⁷ |
| pcTG35/DOPE | 5 | 3,3 x 10⁷ | 6,8 x 10⁷ | 4,3 x 10⁵ | 1,0 x 10⁸ |
| pcTG35 | 10 | 1,2 x 10⁸ | 7,6 x 10⁶ | 1,8 x 10⁷ | 6,2 x 10⁸ |
| pcTG35 | 5 | 1,6 x 10⁸ | 6,6 x 10⁷ | 3,4 x 10⁸ | 1,7 x 10⁸ |

| | | | | | |
|---|---|---|---|---|---|
| 1 :Rapport entre les charges positives du lipide cationique et les charges négatives de l'ADN. | | | | | |
| 2 :ADN libre entre 0 et 270 unités de lumière relative. | | | | | |

L'expression de la luciférase (RLU/min) rapportée en mg de protéines donne les valeurs suivantes :
pcTG35 R +/- 10 (A549) 3,4 x 10¹⁰ RLU/min/mg protéine
pcTG35 R +/- 5 (A549) 2,8 x 10¹⁰ RLU/min/mg protéine
pcTG35/DOPEv R +/- 5 (A549 + sérum) 1,0 x 10¹⁰ RLU/min/mg protéine.

Les composés pcTG20 et pcTG35 sont capables de transfecter efficacement les deux types de cellules testées lorsqu'ils son complexés à l'ADN plasmidique. On notera que certaines formulations donnent des résultats plus élevés en présence de sérum et que le sérum n'a pas d'effet inhibiteur sur ce type de préparation.

De la même façon, des analyses de transfection *in vitro* réalisées sur des cellules A549 ont permis de mettre en évidence a) que le composé pcTG90 complexé à un plasmide pTG11033 permet d'observer une transfection efficace dans des conditions similaires à celles décrites précédemment en absence ou en présence de DOPE, en absence ou en présence de sérum et b) de vérifier que la DOPE pouvait être substituée par un autre adjuvant tel que par exemple la 1,2-di-stéaroyl sn glycéro phospho éthanolamine (Avanti 850715), la 1,2-di-phytanoyl sn glycéro 3 phospho éthanolamine (Avanti 850402), la 1,2-di-myristoyl sn glycéro 3 phospho éthanolamine (Avanti 850745), la 1,2-di-lauroyl sn glycéro 3 phospho éthanolamine (Avanti 850702), la 1,2-di-palmitoyl sn glycéro 3 phospho éthanolamine (Avanti 850705), la 1,2-di-élaidoyl sn glycéro 3 phospho éthanolamine (Avanti 850725), la 1,2-di-palmitoleyl sn glycéro 3 phospho éthanolamine (Avanti 850706) ou la 1,2-di-linoleoyl sn glycéro 3 phospho éthanolamine (Avanti 850755).

### L . Analyse de la taille des particules (selon le protocole décrit en F).

Les analyses conduites par PCS montrent que la taille des particules et leur aggrégation dépendent fortement du rapport de charge, de la structure du lipide ainsi que de la présence ou absence de DOPE.

Des complexes stables de 100 à 200 nm ont été obtenus de manière reproductible pour un rapport de charge de 10, lorsque le glycérolipide cationique est en excés par rapport à l'ADN (voir Figures 2). Les résultats montrent que seuls les complexes renfermant un glycérolipide C18 (pcTG19, pcTG36) présentent un problème de suspension, donnant lieu à de grands complexes dont la taille est variable. Au contraire, les lipides de type oleoyl renfermant 4 ou 5 charges (pcTG35, pcTG56) permettent d'obtenir des complexes d'environ 200 nm pour un rapport de charge de 5. Par ailleurs, nous avons montré qu'une quantité équimolaire de DOPE accroit légèrement la taille des particules et diminue la tendance qu'ont les complexes à s'aggréger à un faible ratio de charge (voir Figures 2A et 2B). La DOPE permet en outre d'obtenir un effet stabilisant pour les complexes renfermant des glycérolipides C-14 ou C-16 avec trois groupes amine (Figure 2A) ; cet effet n'est pas observé pour les composés homologues renfermant quatre fonctions amines (Figure 2B). La tendance des complexes à s'aggréger augmente considérablement pour un rapport de charge de 5 suggérant que les répulsions de charges est un facteur déterminant pour éviter le phénomène d'aggrégation. La comparaison des complexes renfermant des glycérolipides portant des acides gras de longueur croissante présentent des différences mineures entre les dérivés C-14, C-16 et oleoyl pour un rapport de charge de 10, en particulier dans le cas des glycérolipides renfermant 4 groupes amines. Le nombre de groupes amines au niveau de la tête polaire des glycérolipides contenant des oleoyles présente un léger effet sur la taille des complexes (figure 2C).

Il convient en outre de noter que les résultats observés par cette technique de mesure de la taille des complexes ont été confirmés par microscopie électronique.

### M - Injection intraveineuse de complexes selon l'invention.

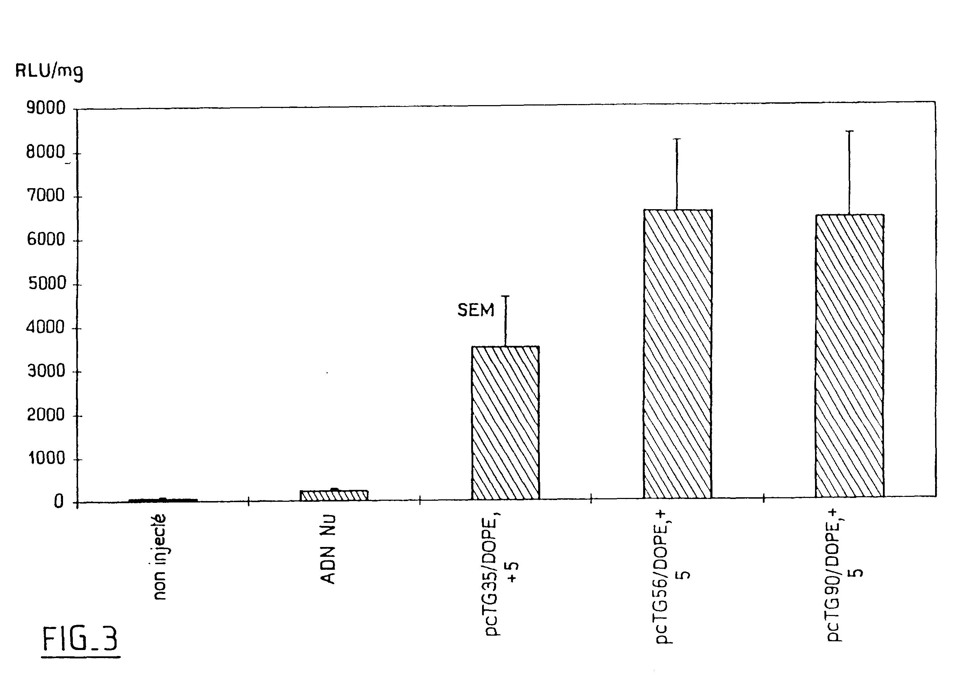

Les résultats sont résumés sur la figure 3. Des complexes selon l'invention ont été synthétisés selon les méthodes décrites précédemment à partir des glycérolipides pcTG35, pcTG56 et pcTG90, en présence d'une quantité équimolaire de DOPE, à un ratio de charge fixe de 5, en utilisant un plasmide contenant le gène de la luciférase pTG11033 (demande de brevet français n° 97/08267).

Les souris utilisées sont des souris femelles C57BL/6 de 9 à il semaines. Les injections intraveineuses sont effectuées dans la queue après désinfection de la peau à l'éthanol 70%. Le volume injecté est 200µl et la concentration en ADN est 0.24 mg/ml (concentration en lipides : 10 mg/ml).

Deux jours après les injections, les souris sont sacrifiées. Après extraction, les tissus sont congelés dans de l'azote liquide et conservés à -80°C. Afin de mesurer l'activité luciférase, les tissus sont broyés mécaniquement à l'aide d'un pilon dans un mortier placé sur la carboglace. 500µl ou 200µl d'un tampon de lyse (Promega) sont ajoutés aux débris de tissus obtenus à partir des poumons ou de la trachée, respectivement, et soumis à trois étapes de congélation/décongélation. Les débris cellulaires sont éliminés par centifugation et l'activité luciférase (en RLU/min, Unité de lumière relative par minute) est mesurée sur 20 µl de surnageant conformément aux instructions du fournisseur (Promega) en ajoutant 100 µl de réactif et en mesurant l'activité par luminescence. L'activité luciférase mesurée est standardisée par rapport à la quantité protéique à l'aide d'une gamme étalon réalisée à partir de luciférase disponible dans le commerce (Promega). La quantité de protéine totale est, par ailleurs, déterminée par la méthode colorimétrique d'acide bicinchoninique BCA (Smith et al., 1985, Anal. Biochem., 150, 76-85 Pierce) à partir d'un aliquote de surnageant. Ceci permet d'exprimer l'activité luciférase en RLU par milligramme de protéine extraite des tissus.

Les résultats montrent que l'expression du gène rapporteur luciférase dans les poumons après injection intraveineuse de complexes renfermant l'un des trois glycérolipides indiqués précédemment en présence de DOPE est nettement améliorée par rapport à l'injection de l'ADN seul d'un facteur environ 15 à 30 fois. Les valeurs indiquées sont des valeurs moyennes obtenues à partir de 2 à 6 souris injectées.

### N - Synthèse de glycérolipides fluorés pcTG69 à pcTG72.

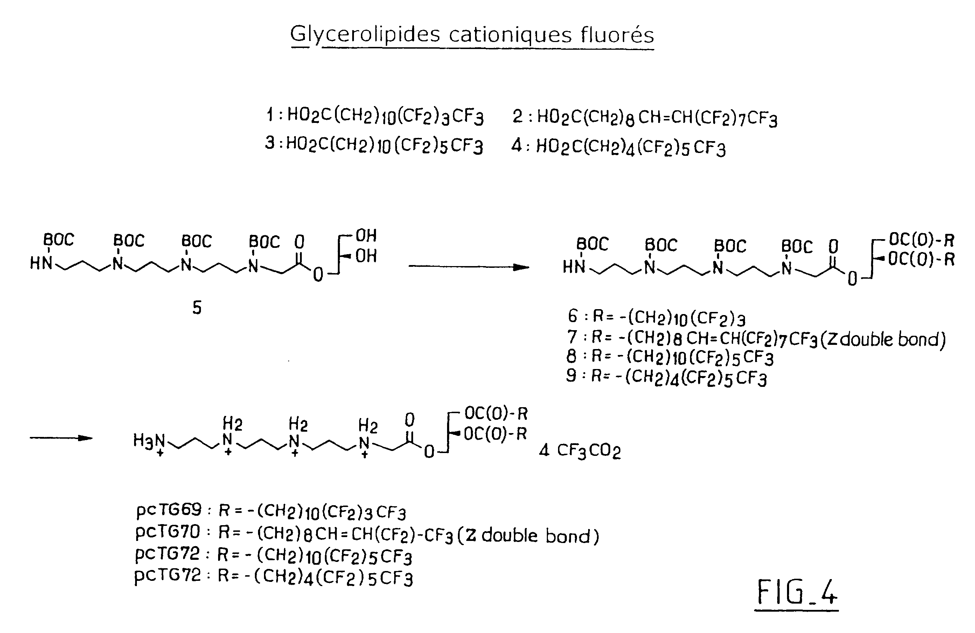

### 1. Synthèse des triesters (voir Figure 4)

Triester 6 : 232 mg (1,12 mmole) de dicyclohexylcarbodiimide dans 1 ml de dichlorométhane sont ajoutés à une solution de dihydroxyester 5 (270 mg, 0,37 mmole), d'acide fluoré 1 (454 mg, 1,12 mmole) et de 4-(diméthylamino)pyridine (5 mg, 0,038 mmole) dans 4 ml de dichlorométhane. La réaction est réalisée sous agitation pendant 16 heures à température ambiante. Le précipité de dicyclohexylurée est éliminé par filtration et le filtrat est concentré sous vide et soumis à une chromatographie sur une colonne de gel de silice (éluant : éther:hexane, 4:6, v:v) afin d'obtenir le triester 6 (275 mg, 48%) sous la forme d'un liquide visqueux. ¹H NMR (200 MHz, CDCl₃) : δ 5.25 (m, 1 H, >CHOC(O)-), 4.40-4.08 (m, 4 H, -CH₂-OC(O)-), 3.95 and 3.89 (2 m, 2 H, -N(BOC)-CH₂-CO₂-), 3.35-3.00 (m, 12 H, -CH₂-N(BOC)-), 2.31 (t, J = 7.5 Hz, 4 H, -CH₂-CO₂-), 2.20-1.88 (m, 4 H, -CH₂-CF₂-), 1.82-1.50 (m, 10 H, -CH₂-), 1.45, 1.44, 1.43, and 1.41 (4 s, 36 H, t-Bu-), 1.29 (br s, 28 H, -CH₂-). ¹⁹F NMR (376 MHz, CDCl₃) : d -81.6, -115.1, -125.0, -126.5.

Triester 7 : Selon un protocole identique à celui décrit pour le triester 6, le triester 7 (220 mg, 49%) est obtenu à partir du dihydroxyester 5 (170 mg, 0,236 mmole) et de l'acide fluoré 2 (426 mg, 0,707 mmole).
¹H NMR (200 MHz, CDCl₃) δ 6.40 (dtt, J = 15.6, 6.9, 2.2 Hz, 2 H, =CH-CF₂-), 5.59 (dt, J = 15.6, 12.3 Hz, 2 H, =CH-CH₂-), 5.25 (m, 1 H, >CH-OC(O)-), 4.40-4.05 (m, 4 H, -CH₂-OC(O)-), 3.96 and 3.89 (2 m, 2 H, -N(BOC)-CH₂-CO₂-), 3.35-3.00 (m, 12 H, -CH₂-N(BOC)-), 2.31 (t, J = 7.2 Hz, 4 H, -CH₂-CO₂-), 2.20 (m, 4 H, allylic H), 1.80-1.50 (m, 10 H, -CH₂-), 1.45, 1.44, 1.43, and 1.41 (4 s, 36 H, *t*-Bu-), 1.30 (br s, 20 H, -CH₂-). ¹⁹F NMR (376 MHz, CDCl₃) : d -81.3, -111.6, -121.9, -122.4, -123.2, -123.9, - 126.6.

Triester 8 : Selon un protocole identique à celui décrit pour le triester 6, le triester 8 (230 mg, 47%) est obtenu à partir du dihydroxyester 5 (210 mg, 0,291 mmole) et de l'acide fluoré 3 (441 mg, 0,874 mmole).
¹H NMR (200 MHz, CDCl₃) : δ 5.25 (m, 1 H, >CH-OC(O)-), 4.40-4.10 (m, 4 H, -CH₂-OC(O)-), 3.95 and 3.89 (2 m, 2 H, -N(BOC)-CH₂-CO₂-), 3.35-3.00 (m, 12 H, -CH₂-N(BOC)-), 2.31 (t, J = 7.5 Hz, 4 H, -CH₂-CO₂-), 2.20-1.90 (m, 4 H, -CH₂-CF₂-), 1.82-1.50 (m, 10 H, -CH₂-), 1.45, 1.44, 1.43, and 1.41 (4 s, 36 H, t-Bu-), 1.29 (br s, 28 H, -CH₂-) . ¹⁹F NMR (376 MHz, CDCl₃) : d -81.3, -114.9, -122.4, -123.2, -124.0, -126.6.

Triester 9 : Selon un protocole identique à celui décrit pour le triester 6, le triester 9 (165 mg, 49%) est obtenu à partir du dihydroxyester 5 (160 mg, 0,222 mmole) et de l'acide fluoré 4 (280 mg, 0,666 mmole).
¹H NMR (200 MHz, CDCl₃) : δ 5.25 (m, 1 H, >CH-OC(O)-), 4.45-4.08 (m, 4 H, -CH₂-OC(O)-), 3.95 and 3.89 (2 m, 2 H, -N(BOC)-CH₂-CO₂-), 3.35-3.00 (m, 12 H, -CH₂-N(BOC)-), 2.37 (m, 4 H, -CH₂-CO₂-), 2.26-1.95 (m, 4 H, -CH₂-CF₂-), 1.85-1.55 (m, 14 H, -CH₂-), 1.45, 1.44, 1.43, and 1.41 (4 s, 36 H, *t*-Bu-). ¹⁹F NMR (376 MHz, CDCl₃) : d-81.3, -114.9, -122.4, -123.4, -124.1, -126.7.

### 2. Synthèse du pcTG69

Le triester 6 (120 mg, 0,079 mmole) dans 1 ml de dichlorométhane est traité 3 heures avec 16 ml d'une solution d'acide trifluoroacétique et dichlorométhane (1:1, v:v) à 0°C. 100 ml d'hexane sont ajoutés et le mélange est évaporé sous vide afin d'obtenir un film qui est repris dans de l'éther distillé. Après filtration, on obtient une poudre blanche qui est lavée à l'éther et séchée sous vide pour obtenir le composé pcTG69 (115 mg, 94%), Mp=205°C.

### 3. Synthèse du pcTG70

Le glycérolipide pcTG70 (185 mg, 86%) est obtenu sous la forme d'une poudre blanche à partir du triester 7 (210 mg, 0,111 mmole) selon un procédé identique à celui décrit pour le pcTG69. Mp=210°C.

### 4. Synthèse du pcTG71

Le glycérolipide pcTG71 (141 mg, 90%) est obtenu sous la forme d'une poudre blanche à partir du triester 8 (150 mg, 0,089 mmole) selon un procédé identique à celui décrit pour le pcTG69. Mp=220°C.

### 5. Synthèse du pcTG72

Le glycérolipide pcTG72 (87 mg, 92%) est obtenu sous la forme d'une poudre blanche à partir du triester 9 (90 mg, 0,06 mmole) selon un procédé identique à celui décrit pour le pcTG69. Mp=220°C.

Ces composés fluorilés correspondent à l'armature du pcTG35 (4 groupes aminés) avec une chaîne polycarbonée de taille variable (C11, C15, C17 ou C19 insaturée). Ils ont été synthétisés selon la méthode d'injection à l'éthanol.

### O. Transfection in vitro des complexes renfermant les composés fluorilés.

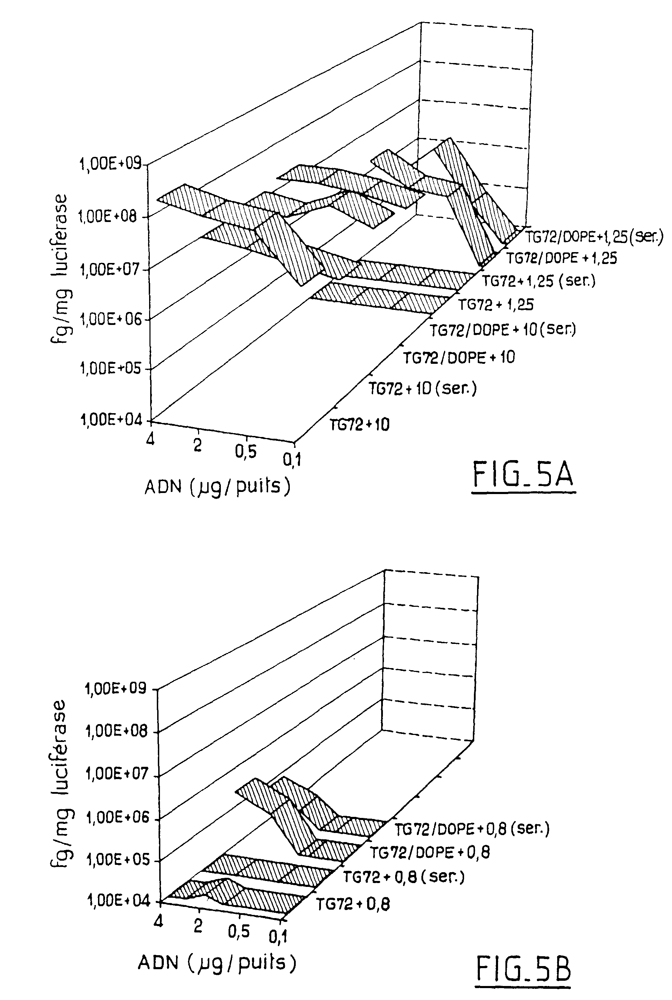

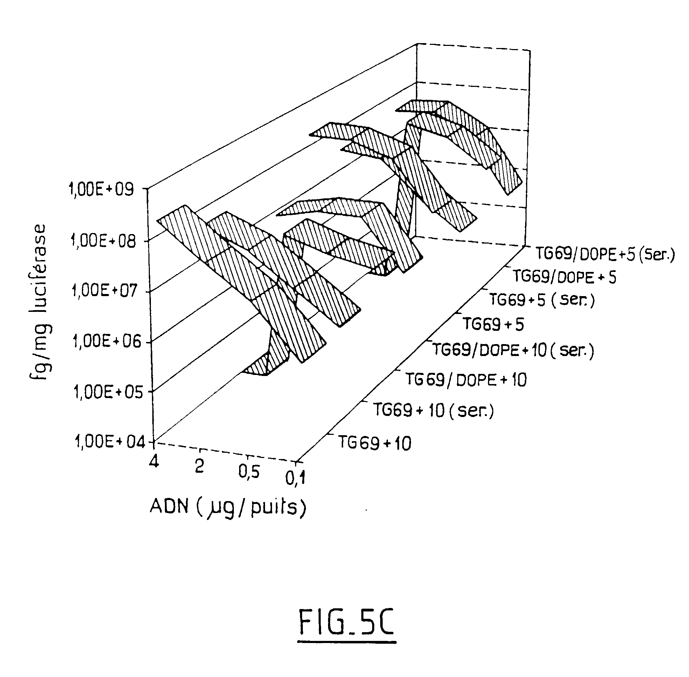

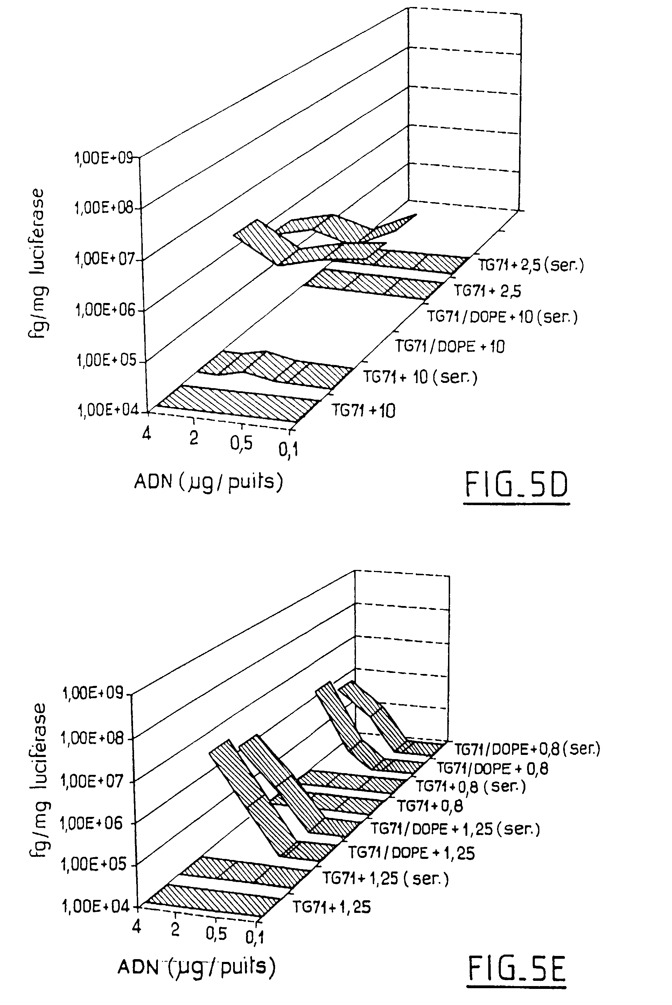

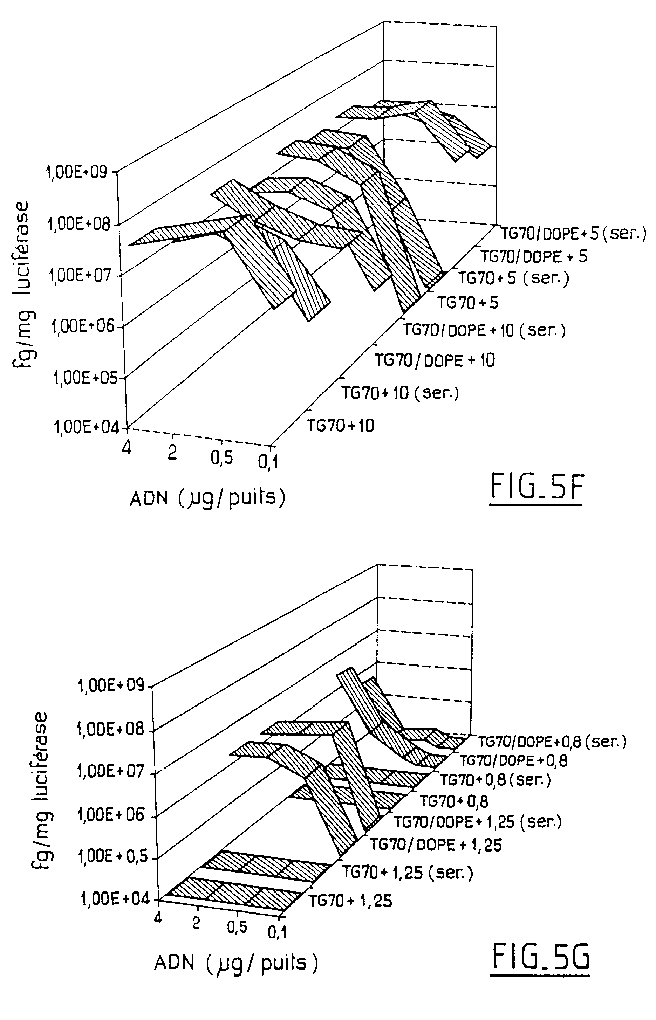

L'efficacité des complexes formés selon la méthode précédemment décrite avec les composés fluorés (voir exemple N)a été étudiée sur une culture de cellules A549 selon la technique décrite à l'exemple I. Les rapports de charges testés sont choisis parmi les rapports 10, 5, 2.5, 1.25 et 0.8 (voir Figures 5A à G). Les essais ont également été réalisés en absence ou en présence (indiqué dans les figures par ser.) de sérum ou de DOPE, et pour différentes quantités d'ADN (0.1, 0.5, 2 ou 4 µg). Les résultats (figures 5 A à G) montrent que:
- le pcTG69 (Figure 5C) permet d'observer, dans les conditions testées, des taux d'expression de la luciférase comparables à ceux obtenus avec des composés analogues non fluorés de C14 ou C16. Par ailleurs, d'autres résultats, non montrés sur la figure 5, ont montré que les meilleurs résultats étaient obtenus avec le pcTG69 avec un rapport de charges de 2.5, en présence ou en absence de DOPE ou de sérum,
- le pcTG72 (Figures 5A et B) permet d'obtenir des taux de transfection satisfaisants pour un rapport de charges de 10, en présence ou en l'absence de DOPE ou de sérum ; par contre, pour un rapport de charge de 1.25 ou 0.8, on constate que la présence de DOPE est un facteur permettant d'améliorer le taux de transfection, notamment en présence de quantités d'ADN supérieures à 0.1 µg,
- le pcTG71 (Figures 5D et E) ne permet d'obtenir des taux de transfection convenables qu'en présence de DOPE, pour des rapports de charge de 1.25, 0.8 ou 10, en particulier pour des quantités d'ADN supérieures à 0.5 µg. Dans les conditions testées, les transfections en présence de DOPE ne semblent pas affectées par la présence de sérum dans le milieu.
- le pcTG70 (Figures 5F et G) : Pour des rapports de charges de 10 ou 5, les taus de transfection observés sont efficaces, en présence ou en absence de DOPE ou de sérum. Par contre, pour des rapports de charges plus faibles (1.25 ou 0.8), la présence de DOPE semble requise.

## Revendications

1. Composé de formule I : 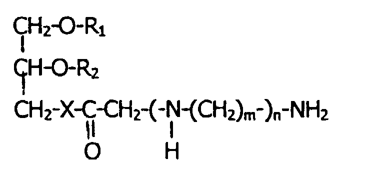 dans laquelle:
R₁, R₂, identiques ou différents sont des radicaux C₆-C₂₃ alkyle ou alcényle, linéaires ou ramifiés ou des radicaux -C(=O)-(C₆-C₂₃) alkyle ou -C(=O)-(C₆-C₂₃) alcényle, linéaires ou ramifiés,
X est l'atome d'oxygène ou un radical amino -NR₃, R₃ étant un atome d'hydrogène ou un radical alkyle de 1 à 4 atomes de carbone,
n est un nombre entier positif de 1 à 6,
m est un nombre entier positif de 1 à 6, et lorsque n > 1, m peut être identique ou différent.

2. Composé selon la revendication 1, caractérisé en ce que R₁, R₂, identiques ou différents sont des radicaux -C(=O) alkyle linéaires ou -C(=O) alcényle linéaires.

3. Composé selon la revendication 1 ou 2, caractérisé en ce que R₁, R₂, identiques ou différents sont des radicaux -C(=O) alkyle ou (C(=O) alcényle, comprenant de 12 à 20 atomes de carbone.

4. Composé selon l'une des revendications 1 à 3, caractérisé en ce que n est un nombre entier choisi parmi les nombres 2, 3 ou 4.

5. Composé selon l'une des revendications 1 à 4, caractérisé en ce que m est un nombre entier choisi parmi les nombres 2, 3 ou 4.

6. Composé selon l'une des revendications 1 à 5, caractérisé en ce qu'il est en outre conjugué à un ou plusieurs éléments de ciblage par l'intermédiaire d'au moins a) un des atomes de carbone, en particulier choisis parmi ceux présents sur les groupements R₁, R₂ et/ou R₃ ou b)un des atomes d'azote secondaire ou primaire de la chaîne polyamine.

7. Composé selon la revendication 6, caractérisé en ce que ledit élément de ciblage est choisi parmi le groupe consistant en tout ou partie de sucres, de peptides, d'oligonucléotides, de lipides, d'hormones, de vitamines, d'antigènes, d'anticorps, de ligands spécifiques de récepteurs membranaires, de ligands susceptibles de réagir avec un anti-ligand, de peptides fusogèniques, de peptides de localisation nucléaire, ou d'une combinaison de tels composés.

8. Composé selon l'une des revendications 1 à 7, caractérisé en ce qu'il est sous forme cationique.

9. Composé selon la revendication 8 sous forme cationique, caractérisé en ce qu'une ou plusieurs amines sont substituées par un (des) radicaux méthyle(s) ou éthyle(s).

10. Composé selon les revendications 8 ou 9 caractérisé en ce qu'il comporte de 2 à 7 charges positives, plus particulièrement de 3 à 5, et préférentiellement 5.

11. Composé selon l'une des revendications 8 à 10 caractérisé en ce qu'il est choisi dans le groupe constitué par les composés de formule suivante :

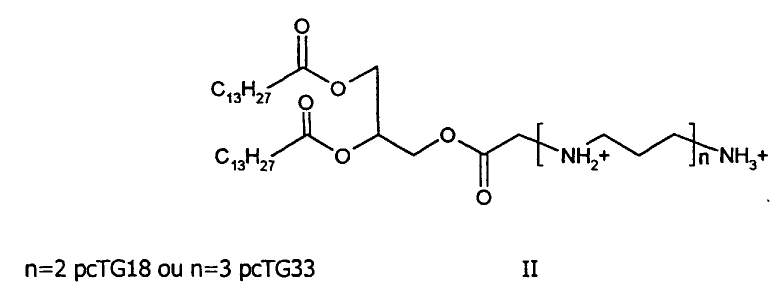

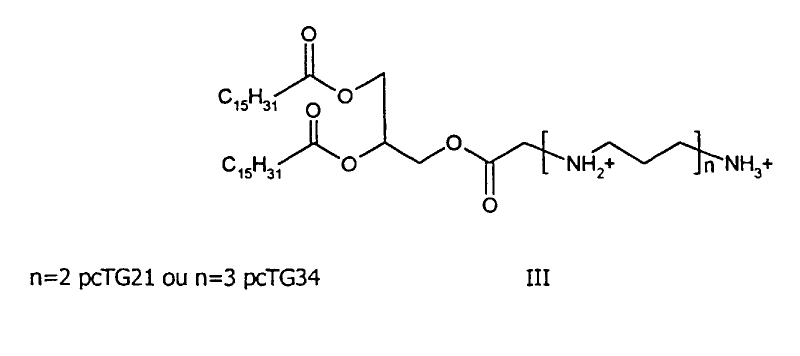

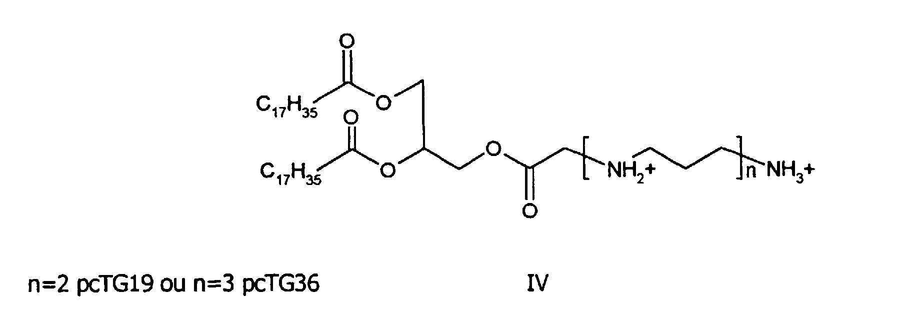

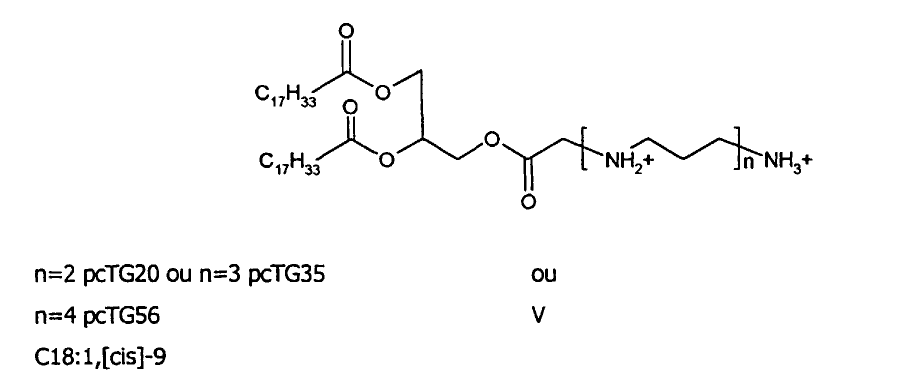

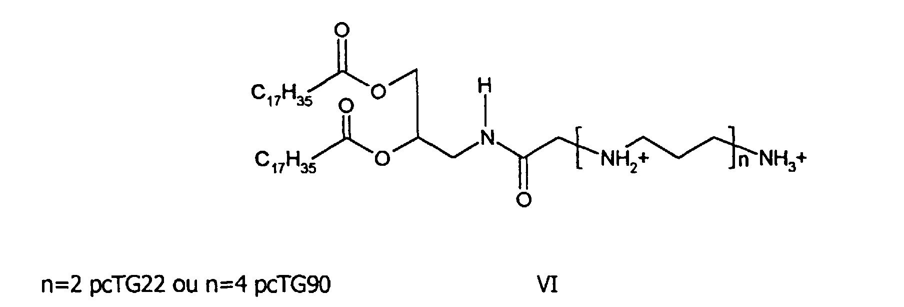

12. Composition caractérisée en ce qu'elle comprend au moins un composé selon l'une des revendications précédentes et éventuellement au moins un adjuvant capable notamment d'améliorer la formation de complexe entre undit composé et une substance active.

13. Composition selon la revendication 12 caractérisée en ce que ledit adjuvant est un lipide neutre ou zwitterionique.

14. Composition selon la revendication 13, caractérisée en ce que ledit lipide neutre ou zwitterionique est ou dérive d'un triglycéride, d'un diglycéride, du cholestérol, d'une phosphatidyl éthanolamine (PE), phosphatidylcholine, phosphocholine, sphyngomyéline, céramide ou cérébroside.

15. Composition selon la revendication 14, caractérisée en ce que ledit lipide neutre ou zwitterionique est la dioleylphosphatidyl éthanolamine (DOPE).

16. Composition selon les revendications 12 à 15, caractérisée en ce que le rapport en poids composé/adjuvant est compris entre 0,1 et 10.

17. Complexe comprenant au moins un composé selon l'une des revendications 8 à 11 ou au moins une composition selon l'une des revendications 12 à 16 et au moins une substance active, notamment thérapeutiquement comportant au moins une charge négative.

18. Complexe selon la revendication 17 caractérisé en ce que ladite substance active est choisie parmi les acides nucléiques et les protéines.

19. Complexe selon la revendication 18 caractérisé en ce ladite substance thérapeutiquement active est un acide nucléique choisi parmi le groupe consistant en un cADN, un ADN génomique, un ADN plasmidique, un polynucléotide antisens, un ARN messager, un ARN ribosomique, un ribozyme, un ARN de transfert, ou un ADN codant pour de tels ARN.

20. Complexe selon la revendication 19 caractérisé en ce ledit acide nucléique comprend un gène d'intérêt et des éléments permettant l'expression dudit gène d'intérêt.

21. Complexe selon l'une des revendications 17 à 20 caractérisé en ce qu'il présente une taille inférieure à 500 nm, avantageusement inférieure à 200 nm.

22. Complexe selon l'une des revendications 17 à 21 caractérisé en ce qu'il présente une taille inférieure à 100 nm.

23. Complexe selon l'une des revendications 17 à 22, caractérisé en ce que le rapport entre le nombre de charges positives du ou des composés et/ou compositions cationiques et le nombre de charges négatives de ladite substance active varie de 0,05 à 20, plus particulièrement de 0,1 à 15, et de préférence de 5 à 10.

24. Procédé de préparation d'un complexe selon les revendications 17 à 23 caractérisé en ce que l'on met en présence un ou plusieurs composés selon l'une des revendications 8 à 11 et/ou au moins une composition selon l'une des revendications 12 à 16 avec une ou plusieurs substances actives comportant au moins une charge négative et en ce que l'on récupère ledit complexe, éventuellement après une étape de purification.

25. Procédé de préparation selon la revendication 24, caractérisé en ce que lesdits composés et/ou des compositions sont au préalable mis en solution dans un solvant miscible à l'eau, notamment l'éthanol ou le diméthylsulfoxide ou un mélange des deux.

26. Procédé de préparation selon la revendication 24, caractérisé en ce que lesdits composés et/ou des compositions sont au préalable mis en suspension dans une solution de détergent.

27. Procédé de préparation selon la revendication 26, caractérisé en ce que l'on procède en outre à une étape de purification dudit complexe par dialyse.

28. Procédé de préparation selon la revendication 24 , caractérisé en ce que lesdits composés et/ou des compositions sont au préalable soumis à une étape de sonication/extrusion.

29. Complexe caractérisé en ce qu'il est susceptible d'être obtenu par un procédé selon l'une des revendications 25 à 28.

30. Utilisation d'un composé selon l'une quelconque des revendications 1 à 11, d'une composition selon l'une quelconque des revendications 12 à 16, d'un complexe selon l'une quelconque des revendications 17 à 23, pour la préparation d'une composition pour transférer au moins une substance active, notamment thérapeutiquement, en particulier un acide nucléique, dans des cellules cibles *in vitro, ex vivo ou in vivo,* plus particulièrement *in vivo.*

31. Utilisation selon la revendication 30 caractérisée en ce que ladite cellule cible est une cellule de mammifère.

32. Utilisation selon la revendication 31 caractérisée en ce que ladite cellule cible est sélectionnée parmi une cellule musculaire, une cellule souche hématopoïétique, une cellule des voies aériennes, plus particulièrement une cellule trachéale ou pulmonaire.

33. Procédé de transfert *in vitro* d'une substance thérapeutiquement active dans une cellule cible, caractérisé en ce qu'on met en contact ladite cellule avec un complexe selon l'une quelconque des revendications 17 à 23.

34. Complexe selon l'une des revendications 17 à 23 ou 29, à titre de médicament, à but curatif, préventif ou vaccinal.

35. Complexe selon la revendication 34 pour la mise en oeuvre d'une méthode de traitement thérapeutique consistant à transférer au moins une substance thérapeutiquement active, notamment un acide nucléique, dans des cellules cibles.

36. Complexe selon la revendication 35 caractérisé en ce que ladite cellule cible est une cellule de mammifère.

37. Complexe selon la revendication 36 caractérisé en ce que ladite cellule cible est sélectionnée parmi une cellule musculaire, une cellule souche hématopoïétique, une cellule des voies aériennes, plus particulièrement une cellule trachéale ou pulmonaire, une cellule de l'épithélium respiratoire.

38. Utilisation d'un composé selon l'une quelconque des revendications 1 à 11, d'une composition selon l'une quelconque des revendications 12 à 16, d'un complexe selon l'une quelconque des revendications 17 à 23 pour la préparation d'un médicament à but curatif, préventif ou vaccinal destiné au traitement du corps humain ou animal, notamment par thérapie génique.

39. Utilisation selon la revendication 38, caractérisée en ce que le médicament est destiné à être administré par injection intramusculaire, par inhalation, par injection intratrachéale, par instillation , par aérosolisation, par voie topique ou par voie orale.

40. Préparation pharmaceutique caractérisée en ce qu'elle comprend au moins un complexe selon l'une quelconque des revendications 17 à 23 ou 29.

41. Préparation selon la revendication 40 caractérisée en ce qu'elle comprend en outre au moins un adjuvant capable d'améliorer le pouvoir de transfection dudit complexe.

42. Préparation selon la revendication 41 caractérisée en ce que ledit adjuvant est choisi parmi le groupe consistant en la chloroquine, un composé polaire protique notamment choisi parmi le propylène glycol, le polyéthylène glycol, le glycérol, l'éthanol, le 1-methyl L -2-pyrrolidone ou leurs dérivés, ou un composé polaire aprotique notamment choisi parmi le diméthylsulfoxide (DMSO), le diéthylsulfoxide, le di-n-propylsulfoxide, le diméthylsulfone, le sulfolane, la diméthylformamide, le diméthylacetamide, la tetraméthylurée, l'acétonitrile ou leurs dérivés.

43. Préparation selon l'une des revendications 40 à 42 caractérisée en ce qu'elle comprend en outre un support acceptable d'un point de vue pharmaceutique permettant son administration à l'homme ou l'animal.

44. Cellule transfectée par un complexe selon l'une quelconque des revendications 17 à 23 ou 29 et 35.

45. Composé selon l'une des revendications 1 à 11, composition selon l'une des revendications 12 à 16, complexe selon l'une des revendications 17 à 23 ou 29 caractérisé en ce R₁ et/ou R₂ est fluoré.

46. Composé, composition ou complexe selon la revendication 45 caractérisé en ce que le nombre d'atomes de carbone fluorés sur R₁ et/ou R₂ peut varier de 1 a 12, plus particulièrement de 4 à 8.

47. Composé, composition ou complexe selon la revendication 45 caractérisé en ce que R₁ et/ou R₂ sont des radicaux alkyle de 15 carbones et en ce que le nombre d'atomes de carbone fluorés sur les chaînes R₁ et/ou R₂ est 4.

## Patentansprüche

1. Verbindung der Formel I: 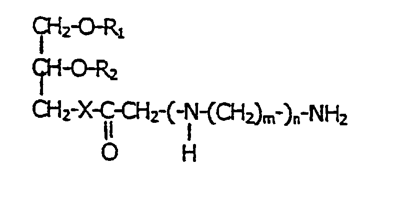 wobei:
R₁, R₂, gleich oder verschieden, lineare oder verzweigte (C₆-C₂₃)Alkyl- oder Alkenylreste oder lineare oder verzweigte -C(=-O)-(C₆-C₂₃)Alkyl- oder -C(=O)-(C₆-C₂₃)Alkenylreste sind,
X ein Sauerstoffatom oder ein Aminorest -NR₃ ist, wobei R₃ ein Wasserstoffatom oder ein Alkylrest mit 1 bis 4 Kohlenstoffatomen ist,
n eine ganze positive Zahl von 1 bis 6 ist,
m eine ganze positive Zahl von 1 bis 6 ist, und wenn n größer 1, m gleich oder verschieden sein kann.

2. Verbindung nach Anspruch 1, dadurch charakterisiert, daß R₁, R₂, gleich oder verschieden, lineare -C(=O)Alkylreste oder lineare -C(=O)Alkenykeste sind.

3. Verbindung nach Anspruch 1 oder 2, dadurch charakterisiert, daß R₁, R₂, gleich oder verschieden, -C(=-O)-Alkyl- oder -C(=O)-Alkenylreste sind, die 12 bis 20 Kohlenstoffatome umfassen.

4. Verbindung nach einem der Ansprüche 1 bis 3, dadurch charakterisiert, daß n eine ganze Zahl ist, die aus den Zahlen 2, 3 oder 4 ausgewählt wird.

5. Verbindung nach einem der Ansprüche 1 bis 4, dadurch charakterisiert, daß m eine ganze Zahl ist, die aus den Zahlen 2, 3 oder 4 ausgewählt wird.

6. Verbindung nach einem der Ansprüche 1 bis 5, dadurch charakterisiert, daß sie darüber hinaus an ein oder mehrere Zielelemente durch Zwischenschalten von mindestens a) eines der Kohlenstoffatome, insbesondere ausgewählt aus denen, die in den Gruppen R₁, R₂ und/oder R₃ vorhanden sind, oder b) eines der sekundären oder primären Stickstoffatome der Polyaminkette gebunden sind.

7. Verbindung nach Anspruch 6, dadurch charakterisiert, daß das Zielelement ausgewählt wird aus der Gruppe, die aus ganzen oder Teilen von Zuckern, Peptiden, Oligonukleotiden, Lipiden, Hormonen, Vitaminen, Antigenen, Antikörpern, spezifischen Liganden von Membran-Rezeptoren, zur Reaktion mit einem Anti-Liganden fähigen Liganden, Fusionspeptiden, Peptiden mit Kernlokalisation oder einer Kombination dieser Verbindungen besteht.

8. Verbindung nach einem der Ansprüche 1 bis 7, dadurch charakterisiert, daß sie in kationischer Form vorliegt.

9. Verbindung nach Anspruch 8 in kationischer Form, dadurch charakterisiert, daß eines oder mehrere Amine mit einem (mehreren) Methyl- oder Ethylrest(en) substituiert sind.

10. Verbindung nach den Ansprüchen 8 oder 9, dadurch charakterisiert, daß sie 2 bis 7, genauer 3 bis 5 und bevorzugt 5 positive Ladungen trägt.

11. Verbindung nach einem der Ansprüche 8 bis 10, dadurch charakterisiert, daß sie ausgewählt wird aus der durch die Verbindungen der folgenden Formel gebildeten Gruppe:

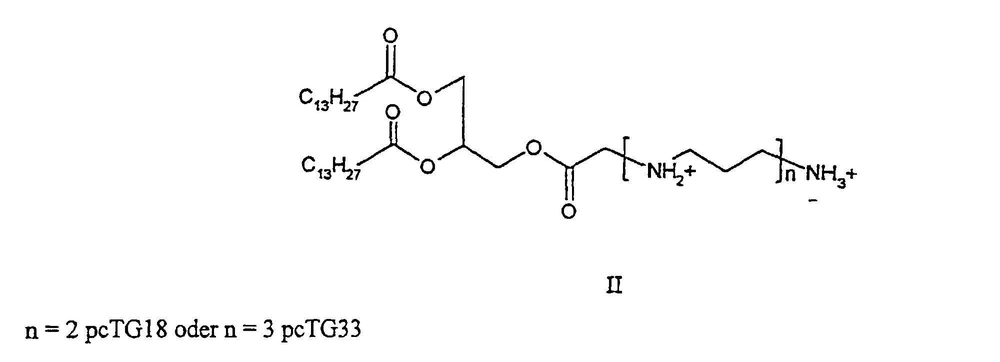

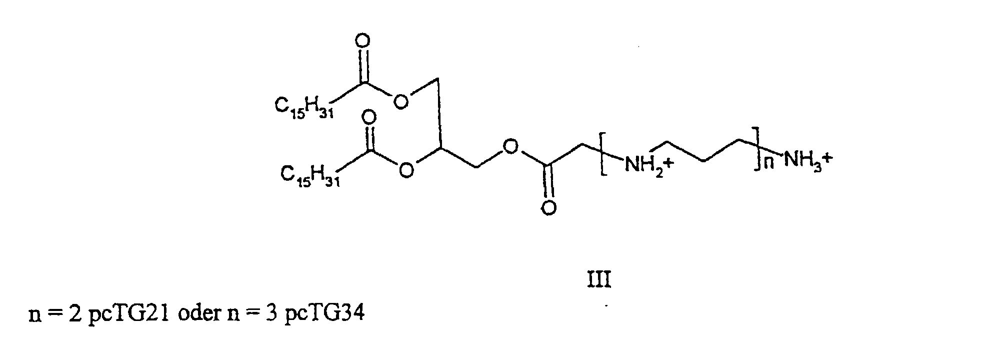

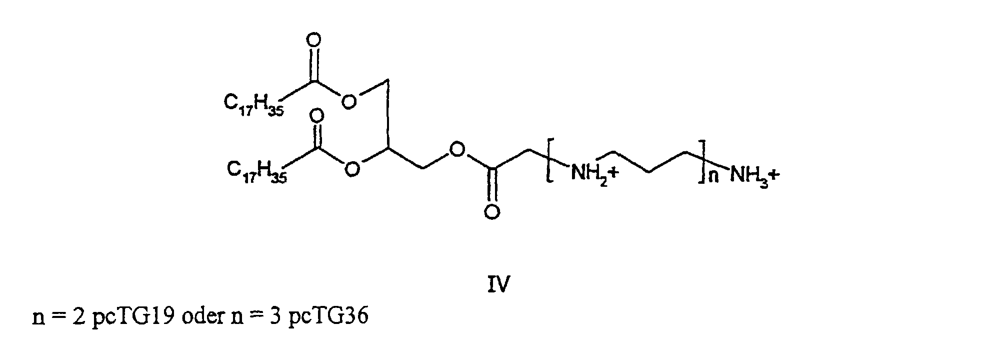

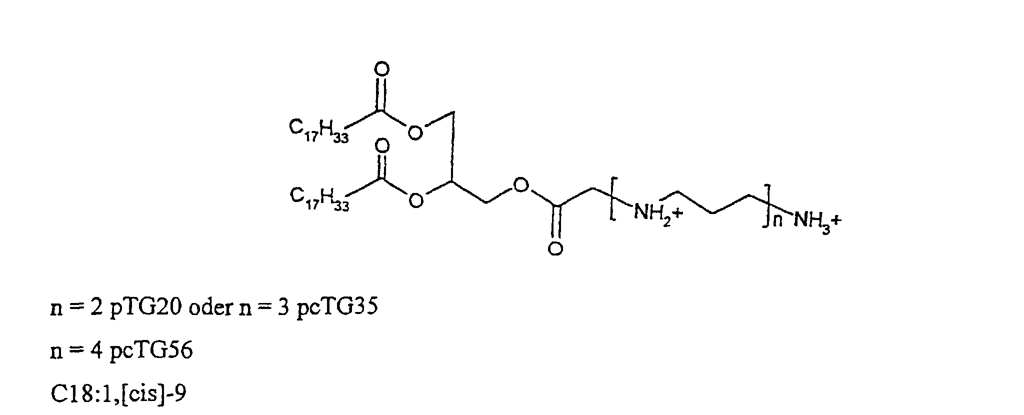

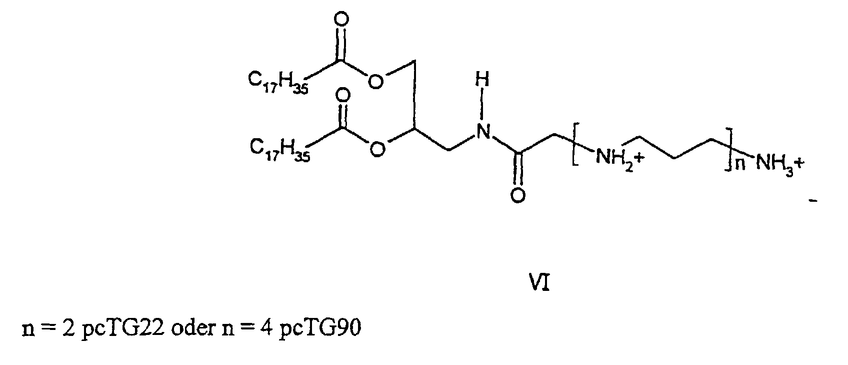

12. Zusammensetzung, dadurch charakterisiert, daß sie mindestens eine Verbindung nach einem der vorstehenden Ansprüche und gegebenenfalls mindestens einen Hilfsstoff umfaßt, der insbesondere zur Verbesserung der Komplexbildung zwischen einer dieser Verbindungen und einer aktiven Substanz fähig ist.

13. Zusammensetzung nach Anspruch 12, dadurch charakterisiert, daß der Hilfsstoff ein neutrales oder zwitterionisches Lipid ist.

14. Zusammensetzung nach Anspruch 13, dadurch charakterisiert, daß das neutrale oder zwitterionische Lipid ein Triglycerid, ein Diglycerid, Cholesterin, ein Phosphatidylethanolamin (PE), Phosphatidylcholin, Phosphocholin, Sphingomyelin, Ceramid oder Cerebrosid ist oder davon abgeleitet ist.

15. Verbindung nach Anspruch 14, dadurch charakterisiert, daß das neutrale oder zwitterionische Lipid Dioleylphosphatidylethanolamin (DOPE) ist.

16. Verbindung nach den Ansprüchen 12 bis 15, dadurch charakterisiert, daß das Gewichtsverhältnis Verbindung/Hilfsstoff zwischen 0,1 und 10 liegt.

17. Komplex, umfassend mindestens eine Verbindung nach einem der Ansprüche 8 bis 11 oder mindestens eine Zusammensetzung nach einem der Ansprüche 12 bis 16 und mindestens eine, insbesondere therapeutisch, aktive Substanz, die mindestens eine negative Ladung trägt.

18. Komplex nach Anspruch 17, dadurch charakterisiert, daß die aktive Substanz ausgewählt wird aus Nukleinsäuren und Proteinen.

19. Komplex nach Anspruch 18, dadurch charakterisiert, daß die therapeutisch wirksame Substanz eine Nukleinsäure ist, die ausgewählt wird aus der aus cDNA, genomischer DNA, plasmidischer DNA, Antisense-Polynukleotiden, Messenger-RNA, ribosomischer RNA, Ribozymen, Transfer-RNA oder für solche RNAs codierender DNA bestehenden Gruppe.

20. Komplex nach Anspruch 19, dadurch charakterisiert, daß die Nukleinsäure ein interessierendes Gen und Elemente umfaßt, die die Expression dieses interessierenden Gens ermöglichen.

21. Komplex nach einem der Ansprüche 17 bis 20, dadurch charakterisiert, daß er eine Größe von weniger als 500 nm, vorteilhafterweise weniger als 200 nm aufweist.

22. Komplex nach einem der Ansprüche 17 bis 21, dadurch charakterisiert, daß er eine Größe von weniger als 100 nm aufweist.

23. Komplex nach einem der Ansprüche 17 bis 22, dadurch charakterisiert, daß das Verhältnis zwischen der Anzahl der positiven Ladungen des oder der kationischen Verbindungen und/oder Zusammensetzungen und der Zahl der negativen Ladungen der aktiven Substanz zwischen 0,05 bis 20, genauer zwischen 0,1 bis 15 und bevorzugt von 5 bis 10 variiert.

24. Verfahren zur Herstellung eines Komplexes nach den Ansprüchen 17 bis 23, dadurch charakterisiert, daß man eine oder mehrere Verbindungen nach einem der Ansprüche 8 bis 11 und/oder mindestens eine Zusammensetzung nach einem der Ansprüche 12 bis 16 in Gegenwart einer oder mehrerer aktiver Substanzen bringt, die mindestens eine negative Ladung umfassen, und daß man den Komplex isoliert, gegebenenfalls nach einem Reinigungsschritt.

25. Verfahren zur Herstellung nach Anspruch 24, dadurch charakterisiert, daß die Verbindungen und/oder Zusammensetzungen zunächst in einem mit Wasser mischbaren Lösungsmittel gelöst werden, insbesondere Ethanol oder Dimethylsulfoxid oder einer Mischung dieser beiden.

26. Herstellungsverfahren nach Anspruch 24, dadurch charakterisiert, daß die Verbindungen und/oder Zusammensetzungen zunächst in einer Detergens-Lösung suspendiert werden.

27. Herstellungsverfahren nach Anspruch 26, dadurch charakterisiert, daß man darüber hinaus einen Reinigungsschritt dieses Komplexes durch Dialyse durchführt.

28. Herstellungsverfahren nach Anspruch 24, dadurch charakterisiert, daß die Verbindungen und/oder Zusammensetzungen zunächst einem Beschallungs/Extrusionsschritt unterworfen werden.

29. Komplex, dadurch charakterisiert, daß er durch ein Verfahren nach einem der Ansprüche 25 bis 28 erhalten werden kann.

30. Verwendung einer Verbindung nach einem der Ansprüche 1 bis 11, einer Zusammensetzung nach einem der Ansprüche 12 bis 16, eines Komplexes nach einem der Ansprüche 17 bis 23 zur Herstellung einer Zusammensetzung zur Überführung von mindestens einer, insbesondere therapeutisch, aktiven Substanz, insbesondere einer Nukleinsäure, in Zielzellen in vitro, ex vivo oder in vivo, insbesondere in vivo.

31. Verwendung nach Anspruch 30, dadurch charakterisiert, daß die Zielzelle eine Säugerzelle ist.

32. Verwendung nach Anspruch 31, dadurch charakterisiert, daß die Zielzelle ausgewählt wird aus einer Muskelzelle, einer blutbildenden Zelle, einer Zelle der Atemwege, insbesondere einer Tracheal- oder Pulmonarzelle.

33. Verfahren zur Überführung in vitro einer therapeutisch aktiven Substanz in eine Zielzelle, dadurch charakterisiert, daß man diese Zelle mit einem Komplex nach einem der Ansprüche 17 bis 23 in Kontakt bringt.

34. Komplex nach einem der Ansprüche 17 bis 23 oder 29 als Medikament mit kurativem, präventivem oder Impfziel.

35. Komplex nach Anspruch 34 zur Durchführung einer Methode zur therapeutischen Behandlung, die aus der Übertragung von mindestens einer therapeutisch wirksamen Substanz, insbesondere einer Nukleinsäure, in Zielzellen besteht.

36. Komplex nach Anspruch 35, dadurch charakterisiert, daß die Zielzelle eine Säugerzelle ist.

37. Komplex nach Anspruch 36, dadurch charakterisiert, daß die Zielzelle ausgewählt wird aus einer Muskelzelle, einer blutbildenden Zelle, einer Zelle der Atemwege, insbesondere einer Tracheal- oder Pulmonarzelle, einer Zelle des Atemwegsepithels.

38. Verwendung einer Verbindung nach einem der Ansprüche 1 bis 11, einer Zusammensetzung nach einem der Ansprüche 12 bis 16, eines Komplexes nach einem der Ansprüche 17 bis 23 zur Herstellung eines Medikaments mit kurativem, präventivem oder Impfziel, das bestimmt ist zur Behandlung des menschlichen oder tierischen Körpers, insbesondere durch Gentherapie.

39. Verwendung nach Anspruch 38, dadurch charakterisiert, daß das Medikament bestimmt ist zur Verabreichung durch intramuskuläre Injektion, durch Inhalation, durch intratracheale Injektion, durch Einträufeln, durch Aerosolisierung, auf topischem Weg oder auf oralem Weg.

40. Pharmazeutisches Präparat, dadurch charakterisiert, daß es mindestens einen Komplex nach einem der Ansprüche 17 bis 2 3 oder 29 umfaßt.

41. Präparat nach Anspruch 40, dadurch charakterisiert, daß es darüber hinaus mindestens einen Hilfsstoff umfaßt, der die Transfektionskraft dieses Komplexes verbessern kann.

42. Präparat nach Anspruch 41, dadurch charakterisiert, daß der Hilfsstoff ausgewählt wird aus der Gruppe, die besteht aus Chloroquin, einer polaren protischen Verbindung, insbesondere ausgewählt aus Propylenglykol, Polyethylenglykol, Glycerin, Ethanol, 1-Methyl-L-2-pyrrolidon oder deren Derivaten, oder einer polaren aprotischen Verbindung, insbesondere ausgewählt aus Dimethylsulfoxid (DMSO), Diethylsulfoxid, Di-n-propylsolfoxid, Dimethylsulfon, Sulfolan, Dimethylformamid, Dimethylacetamid, Tetramethylharnstoff, Acetonitril oder deren Derivaten.

43. Präparat nach einem der Ansprüche 40 bis 42, dadurch charakterisiert, daß es darüber hinaus einen pharmazeutisch verträglichen Träger umfaßt, der seine Verabreichung an Menschen oder Tiere ermöglicht.

44. Mit einem Komplex nach einem der Ansprüche 17 bis 23 oder 29 und 35 transfizierte Zelle.

45. Verbindung nach einem der Ansprüche 1 bis 11, Zusammensetzung nach einem der Ansprüche 12 bis 16, Komplex nach einem der Ansprüche 17 bis 23 oder 29, dadurch charakterisiert, daß R₁ und/oder R₂ fluoriert ist.

46. Verbindung, Zusammensetzung oder Komplex nach Anspruch 45, dadurch charakterisiert, daß die Anzahl der fluorierten Kohlenstoffatome in R₁ und/oder R₂ von 1 bis 12, insbesondere von 4 bis 8 variieren kann.

47. Verbindung, Zusammensetzung oder Komplex nach Anspruch 45, dadurch charakterisiert, daß R₁ und/oder R₂ Alkylreste mit 15 Kohlenstoffatomen sind und daß die Anzahl der fluorierten Kohlenstoffatome in den Ketten R₁ und/oder R₂ 4 beträgt.

## Claims

1. Compound of formula I : 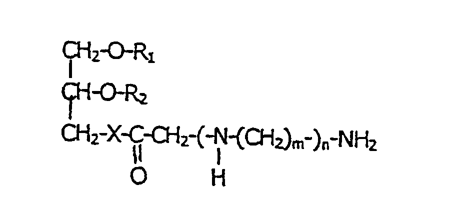 in which:
R₁ and R₂, which are identical or different, are C₆-C₂₃ alkyl or alkenyl radicals which are linear or branched, or radicals -C(=O)-(C₆-C₂₃) alkyl or -C(=O)-(C₆-C₂₃) alkenyl which are linear or branched,
X is an oxygen atom or an amino radical -NR₃, R₃ being a hydrogen atom or an alkyl radical having 1 to 4 carbon atoms,
n is a positive integer from 1 to 6,
m is a positive integer from 1 to 6, and when n > 1, m may be identical or different.

2. Compound according to Claim 1, characterized in that R₁ and R₂, which are identical or different, are linear -C(=O) alkyl or linear -C(=O) alkenyl radicals.

3. Compound according to Claim 1 or 2, characterized in that R₁ and R₂, which are identical or different, are -C(=O) alkyl or -C(=O) alkenyl radicals comprising from 12 to 20 carbon atoms.

4. Compound according to one of Claims 1 to 3, characterized in that n is an integer chosen from the numbers 2, 3 or 4.

5. Compound according to one of Claims 1 to 4, characterized in that m is an integer chosen from the numbers 2, 3 or 4.

6. Compound according to one of Claims 1 to 5, characterized in that it is, in addition, conjugated with one or more targeting components via the intermediacy of at least a) one of the carbon atoms, in particular chosen from those present on the groups R₁, R2 and/or R3 or b) one of the secondary or primary nitrogen atoms of the polyamine chain.

7. Compound according to Claim 6, characterized in that said targeting component is chosen from the group consisting of all or part of sugars, peptides, oligonucleotides, lipids, hormones, vitamins, antigens, antibodies, ligands specific for membrane receptors, ligands capable of reacting with an anti-ligand, fusogenic peptides, nuclear localization peptides, or a combination of such compounds.

8. Compound according to one of Claims 1 to 7, characterized in that it is in a cationic form.

9. Compound according to Claim 8 in a cationic form, characterized in that one or more amines are substituted with (a) methyl or ethyl radical(s).

10. Compound according to Claim 8 or 9, characterized in that it comprises from 2 to 7 positive charges, more particularly from 3 to 5, and preferably 5.

11. Compound according to one of Claims 8 to 10, characterized in that it is chosen from the group consisting of the compounds of the following formula:

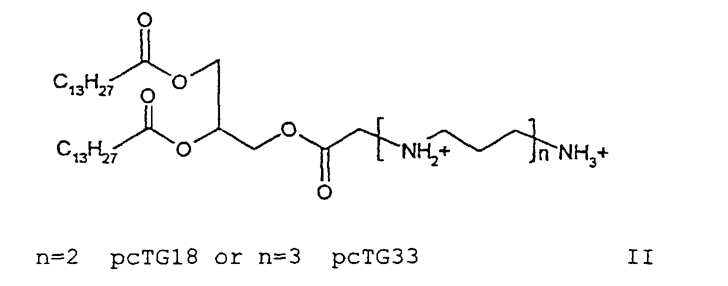

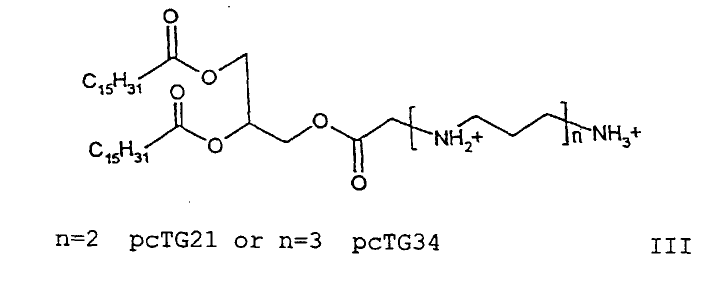

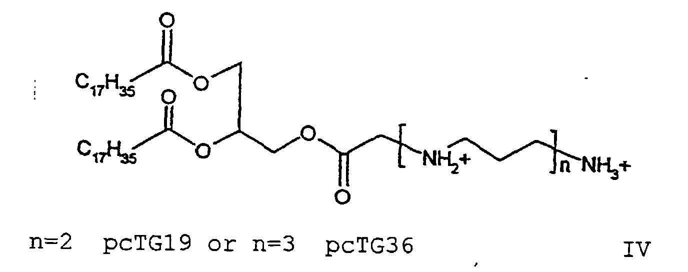

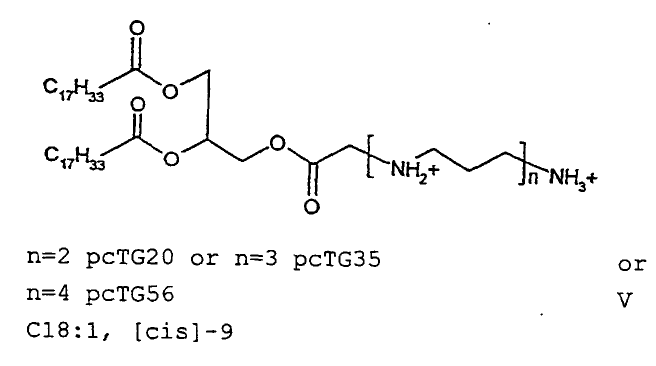

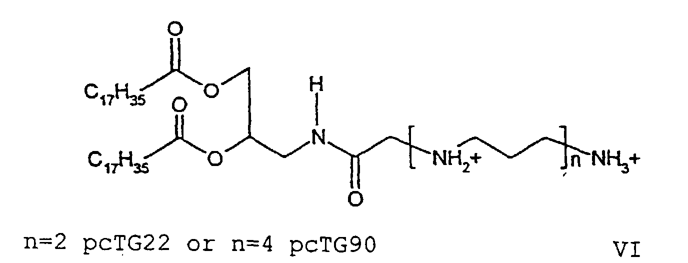

12. Composition characterized in that it comprises at least one compound according to one of the preceding claims and optionally at least one adjuvant capable in particular of enhancing the formation of the complex between said compound and an active substance.

13. Composition according to Claim 12, characterized in that said adjuvant is a neutral or zwitterionic lipid.

14. Composition according to Claim 13, characterized in that said neutral or zwitterionic lipid is or is derived from a triglyceride, a diglyceride, cholesterol, a phosphatidylethanolamine (PE), phosphatidylcholine, phosphocholine, sphyngomyelin, ceramide or cerebroside.

15. Composition according to Claim 14, characterized in that said neutral or zwitterionic lipid is dioleylphosphatidylethanolamine (DOPE).

16. Composition according to Claims 12 to 15, characterized in that the compound/adjuvant weight ratio is between 0.1 and 10.

17. Complex comprising at least one compound according to one of Claims 8 to 11 or at least one composition according to one of Claims 12 to 16 and at least one active substance, in particular a therapeutically active substance, comprising at least one negative charge.

18. Complex according to Claim 17, characterized in that said active substance is chosen from nucleic acids and proteins.

19. Complex according to Claim 18, characterized in that said therapeutically active substance is a nucleic acid chosen from the group consisting of a cDNA, a genomic DNA, a plasmid DNA, an antisense polynucleotide, a messenger RNA, a ribosomal RNA, a ribozyme, a transfer RNA, or a DNA encoding such RNAs.

20. Complex according to Claim 19, characterized in that said nucleic acid comprises a gene of interest and components allowing the expression of said gene of interest.

21. Complex according to one of Claims 17 to 20, characterized in that it has a size of less than 500 nm, advantageously less than 200 nm.

22. Complex according to one of Claims 17 to 21, characterized in that it has a size of less than 100 nm.

23. Complex according to one of Claims 17 to 22, characterized in that the ratio between the number of positive charges of the cationic compound(s) and/or composition(s) and the number of negative charges of said active substance varies from 0.05 to 20, more particularly from 0.1 to 15, and preferably from 5 to 10.

24. Process for preparing a complex according to Claims 17 to 23, characterized in that one or more compounds according to one of Claims 8 to 11 and/or at least one composition according to one of Claims 12 to 16 are brought into contact with one or more active substances comprising at least one negative charge and in that said complex is recovered, optionally after a purification step.

25. Process of preparation according to Claim 24, characterized in that said compounds and/or compositions are dissolved beforehand in a solvent which is miscible with water, in particular ethanol or dimethyl sulphoxide or a mixture of both.

26. Process of preparation according to Claim 24, characterized in that said compounds and/or compositions are suspended beforehand in a detergent solution.

27. Process of preparation according to Claim 26, characterized in that, in addition, a step of purification of said complex by dialysis is carried out.

28. Process of preparation according to Claim 24, characterized in that said compounds and/or compositions are subjected beforehand to a sonication/extrusion step.

29. Complex characterized in that it is capable of being obtained by a process according to one of Claims 25 to 28.

30. Use of a compound according to any one of Claims 1 to 11, of a composition according to any one of Claims 12 to 16, of a complex according to any one of Claims 17 to 23, for the preparation of a composition to transfer at least one active substance, especially a therapeutically active substance, in particular a nucleic acid, into target cells *in vitro, ex vivo* or *in vivo,* more particularly *in vivo.*

31. Use according to Claim 30, characterized in that said target cell is a mammalian cell.

32. Use according to Claim 31, characterized in that said target cell is selected from a muscle cell, a haematopoietic stem cell, a cell of the airways, more particularly a tracheal or pulmonary cell.

33. Process for transferring *in vitro* a therapeutically active substance into a target cell, characterized in that said cell is brought into contact with a complex according to any one of Claims 17 to 23.

34. Complex according to one of Claims 17 to 23 or 29, as a medicament for curative, preventive or vaccinal purposes.

35. Complex according to Claim 34 for carrying out a method of therapeutic treatment consisting in transferring at least one therapeutically active substance, in particular a nucleic acid, into target cells.

36. Complex according to Claim 35, characterized in that said target cell is a mammalian cell.

37. Complex according to Claim 36, characterized in that said target cell is selected from a muscle cell, a haematopoietic stem cell, a cell of the airways, more particularly a tracheal or pulmonary cell, a cell of the respiratory epithelium.

38. Use of a compound according to any one of Claims 1 to 11, of a composition according to any one of Claims 12 to 16, of a complex according to any one of Claims 17 to 23 for the preparation of a medicament for curative, preventive or vaccinal purposes, intended for the treatment of the human or animal body, in particular by gene therapy.

39. Use according to Claim 38, characterized in that the medicament is intended to be administered by intramuscular injection, by inhalation, by intratracheal injection, by instillation, by aerosolization, by the topical route or by the oral route.

40. Pharmaceutical preparation characterized in that it comprises at least one complex according to any one of Claims 17 to 23 or 29.

41. Preparation according to Claim 40, characterized in that it comprises, in addition, at least one adjuvant capable of enhancing the transfecting power of said complex.

42. Preparation according to claim 41, characterized in that said adjuvant is chosen from the group consisting of chloroquine, a protic polar compound chosen in particular from propylene glycol, polyethylene glycol, glycerol, ethanol, 1-methyl-L-2-pyrrolidone or derivatives thereof, or an aprotic polar compound chosen in particular from dimethyl sulphoxide (DMSO), diethyl sulphoxide, di-n-propyl sulphoxide, dimethyl sulphone, sulpholane, dimethylformamide, dimethylacetamide, tetramethylurea, acetonitrile or derivatives thereof.

43. Preparation according to one of claims 40 to 42, characterized in that it comprises, in addition, a pharmaceutically acceptable carrier allowing its administration to humans or animals.

44. Cell transfected with a complex according to any one of Claims 17 to 23 or 29, and 35.

45. Compound according to one of Claims 1 to 11, composition according to one of Claims 12 to 16, complex according to one of Claims 17 to 23 or 29, characterized in that R₁ and/or R₂ is fluorinated.

46. Compound, composition or complex according to Claim 45, characterized in that the number of fluorinated carbon atoms on R₁ and/or R₂ may vary from 1 to 12, more particularly from 4 to 8.

47. Compound, composition or complex according to Claim 45, characterized in that R₁ and/or R₂ are alkyl radicals having 15 carbons and in that the number of fluorinated carbon atoms on the chains R₁ and/or R₂ is 4.
